# EUROPEAN PATENT APPLICATION

(11) **EP 2 457 593 A1**
(43) Date of publication of application: **30.05.2012**
(21) Application number: 10382292.0
(22) Date of filing: 08.11.2010
(51) Int. Cl.: A61K 47/48, C07K 5/08, C07K 5/10, C07K 5/12

(54) **Cyclic RGD peptides of amino acids based on thiazoles or oxazoles as selective antagonists of the alpha-v beta-3 integrin**

(71) Applicant: Lykera Biomed S.A., 08225 Terrasa (ES); Fundació Privada Institut de Recerca Biomèdica, 08028 Barcelona (ES); Universidad de Barcelona, 08028 Barcelona (ES); Fundació Privada Parc Científic de Barcelona, 08028 Barcelona (ES)
(72) Inventor: Mitjans Prat, Francesc, E-08225, TERRASA (ES); Adan Plana, Jaume, E-08225, TERRASA (ES); Calvis Calpe, Carme, E-08225, TERRASA (ES); Albericio Palomera, Fernando, E-08007, BARCELONA (ES); Lavilla Grifols, Rodolfo, E-08860, CASTELLDEFELS (ES); Ruiz Rodriguez, Javier, CH-8093 Zürich ZH (CH); Miguel Sala, Miriam, E-08014, BARCELONA (ES)
(74) Representative: ABG Patentes, S.L.

(57) **Abstract**

The present invention relates to compounds of general formula (I) with selective antagonist activity of the αᵥβ₃ integrin, its production process, and pharmaceutical compositions and conjugates containing them. The invention also covers their medical and diagnostic uses and as carriers of other therapeutic agents.

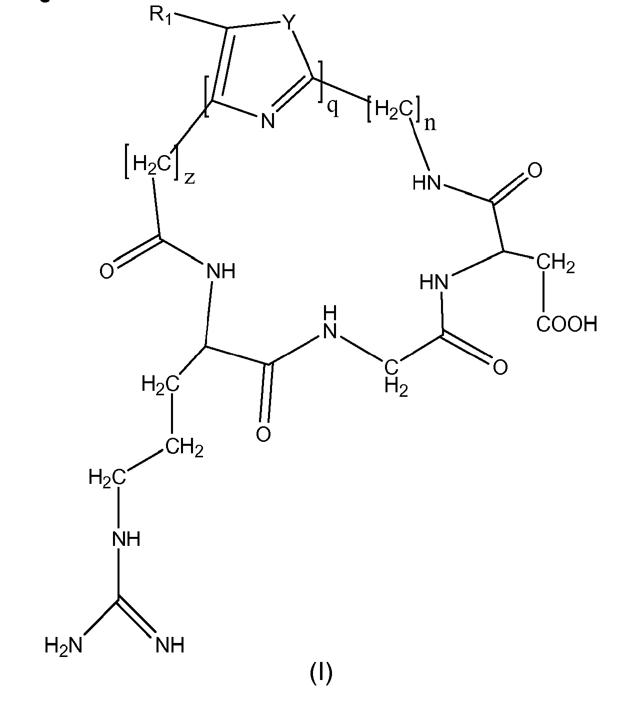

## Description

### TECHNICAL FIELD OF THE INVENTION

The present invention relates to compounds of general formula (I) with selective antagonist activity of the αᵥβ₃ integrin; their production process; pharmaceutical compositions and conjugates containing them; and their medical and diagnostic uses and as carriers of other therapeutic agents.

### BACKGROUND OF THE INVENTION

The interaction between the extracellular matrix and the membrane proteins controls cell adhesion, tissue formation and cross-membrane signalling processes. In this context, the integrins constitute a family of transmembrane heterodimeric receptors that are presented as a promising biological target, due to the important role they perform in many biological functions (Takada Y. et. al Genome Biol 2007, 8:215). Specifically, the integrins of the αᵥβ₃ and αᵥβ₅ subfamilies are directly involved in tumour metastasis and tumour-induced angiogenesis. These two subtypes of integrins are highly expressed in endothelial cells and in many tumour cell lines. Antibodies that block the function of said integrins in cells under culture and also in animal models have demonstrated that the survival and proliferation of endothelial cells depends on the adhesive activity of the αᵥβ₃ and αᵥβ₅ integrins. Since then, antagonists of the αᵥβ₃ and αᵥβ₅ integrins have been designed for the therapeutic treatment of angiogenesis and metastasis (Cai W and Chen X. Anticancer Agents Med Chem 2006, 6:407-428), two of the more serious processes in the development of the cancer disease.

A subgroup of integrins, including αᵥβ₃ and αᵥβ₅ integrins, specifically recognises the Arg-Gly-Asp (RGD) tripeptide sequence in its ligands. Therefore, it is possible to inhibit the function of the integrins blocking their interaction with the RGD sequence in its ligands using analogues of the RGD tripeptide.

To date, many derivatives of cyclic RGD have been synthesized, among which we can highlight the integrin antagonist pentapeptide known as Cilengitide® (currently in phase III clinical testing against glioblastoma) (Prous Science Integrity. Entry number 253338) and those described in Dechantsreiter MA et al. (J Med Chem 1999, 42:3033-3040), as well as other cyclopeptides that include non-natural amino acids (Urman S et al. Angew Chem Int Ed 2007, 46:3976-3978; Belvisi L et al. Org Lett 2001, 3:1001-1004; Haubner R et al. J Am Chem Soc 1996, 118:7881-7891).

Nevertheless, the αᵥβ₅ integrin is also expressed in healthy tissues, so that to carry out a suitable therapy it is necessary to develop integrin antagonist compounds that show selectivity for the αᵥβ₃ integrin over αᵥβ₅ integrin, in this way making it possible to decrease the secondary effects associated to the treatment.

Lohof E et al. (Angew Chem Int Ed 2000, 39:2761-2464) describes various cyclic RGD peptides that contain a carbohydrate, including compounds 1 and 2, which show integrin antagonist activity and selectivity for αᵥβ₃ against αᵥβ₅.

Da Ressurreiçao ASM et al. (Chem Eur J 2009, 15:12184-12188) describes derivatives of cyclic RGD that contain a heterocyclic ring of diketopiperazine and are selective antagonists for the αᵥβ₃ integrin over αᵥβ₅.

Therefore, there is a need in the state of the art to provide new integrin antagonists selective for the αᵥβ₃ integrin over αᵥβ₅.

### SUMMARY OF THE INVENTION

In a first aspect, the invention relates to a compound of general formula (I), its stereoisomers, mixtures thereof and/or its pharmaceutically acceptable salts.

In another aspect, the invention relates to a process for obtaining a compound of general formula (I), its stereoisomers, mixtures thereof or its pharmaceutically acceptable salts comprising the cyclization between the amino-terminal end and the carboxyl-terminal end of a linear peptide selected from the group consisting of:
Arg-Gly-Asp-X
Gly-Asp-X-Arg
Asp-X-Arg-Gly and
X-Arg-Gly-Asp
wherein X is an amino acid based on thiazole and/or oxazole nuclei of formula (IV).

In another aspect, the invention relates to a linear peptide selected from one of the following formulas
Arg-Gly-Asp-X
Gly-Asp-X-Arg
Asp-X-Arg-Gly and
X-Arg-Gly-Asp
wherein X is an amino acid based on thiazole and/or oxazole nuclei of formula (IV) and wherein the residues of Arg and Asp are optionally protected.

In another aspect, the invention relates to a pharmaceutical composition comprising a pharmaceutically effective quantity of at least one compound of general formula (I), its stereoisomers, mixtures thereof and/or its pharmaceutically acceptable salts in accordance with the invention and at least one pharmaceutically acceptable excipient.

In a further aspect, the invention relates to the use of a compound according to the invention for the preparation of a medicament for the prevention and/or the treatment of a disease associated to an undesired cellular proliferation, a disease associated to an undesired angiogenesis, metastasis, restenosis, osteoporosis, an inflammatory disease, proliferative diabetic retinopathy and/or acute renal insufficiency.

In additional aspects, the invention relates to a conjugate comprising a compound according to the invention and a second component selected from the group of
(i) a cytotoxic agent and
(ii) an anti-angiogenic agent
as well as to the use of said conjugates for the preparation of a medicament for the treatment of a disease associated to an undesired cellular proliferation or to an undesired angiogenesis.

In additional aspects, the invention relates to a conjugate comprising a compound according to the invention and a diagnostic imaging agent as well as to the use of said conjugate for the preparation of a diagnostic agent.

In additional aspects, the invention relates to a conjugate comprising a compound according to the invention and a nanocarrier as well as to the use of said conjugates, wherein the nanocarrier contains a cytotoxic agent, an anti-angiogenic agent or a mixture of both, for the preparation of a medicament for the treatment of a disease associated to an undesired cellular proliferation or to an undesired angiogenesis.

In another aspect, the invention relates to a compound according to the invention for the detection of integrins in a sample.

In another aspect, the invention relates to the use of a compound according to the invention or of a conjugate comprising a compound according to the invention and a solid support for the purification or for the detection of integrins in a sample.

### BRIEF DESCRIPTION OF THE FIGURES

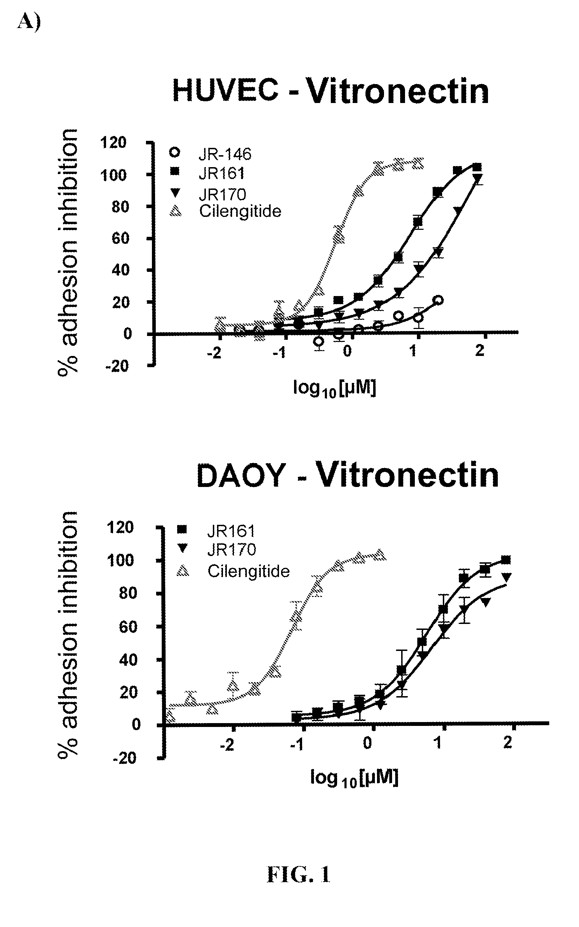

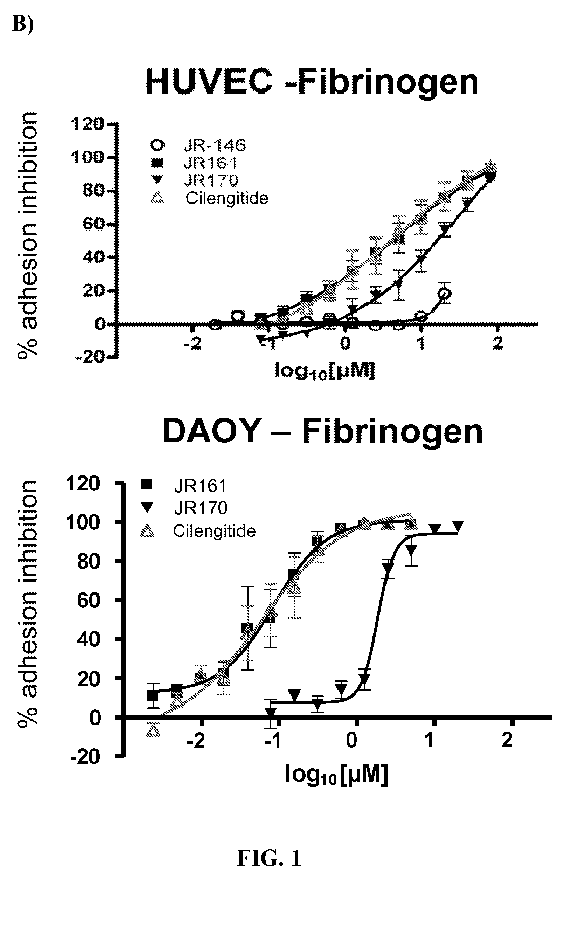

- **Figure 1.**: Cell adhesion inhibition curves for compounds 3 (JR-146), 4 (JR161) and 5 (JR170) and for cilengitide as comparative control in HUVEC and DAOY cell lines. **A)** Using vitronectin (VN) as ligand. **B)** Using fibrinogen (FB) as ligand.

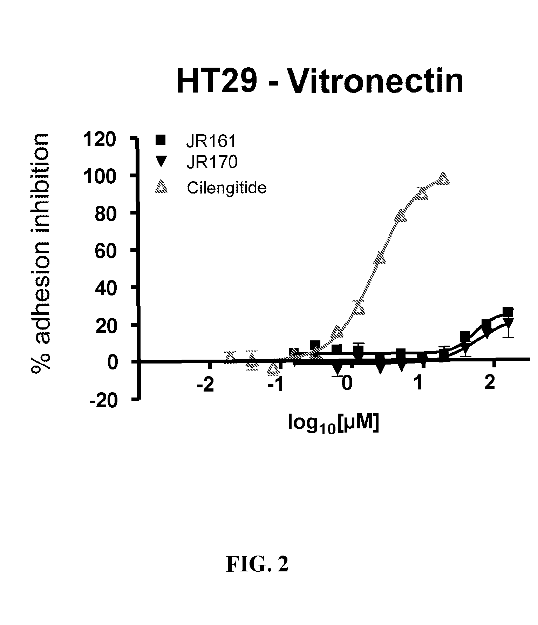

- **Figure 2.**: Cell adhesion inhibition curves for compounds 4 (JR161) and 5 (JR170) and for cilengitide as comparative control in the HT29 cell line using vitronectin (VN) as ligand.

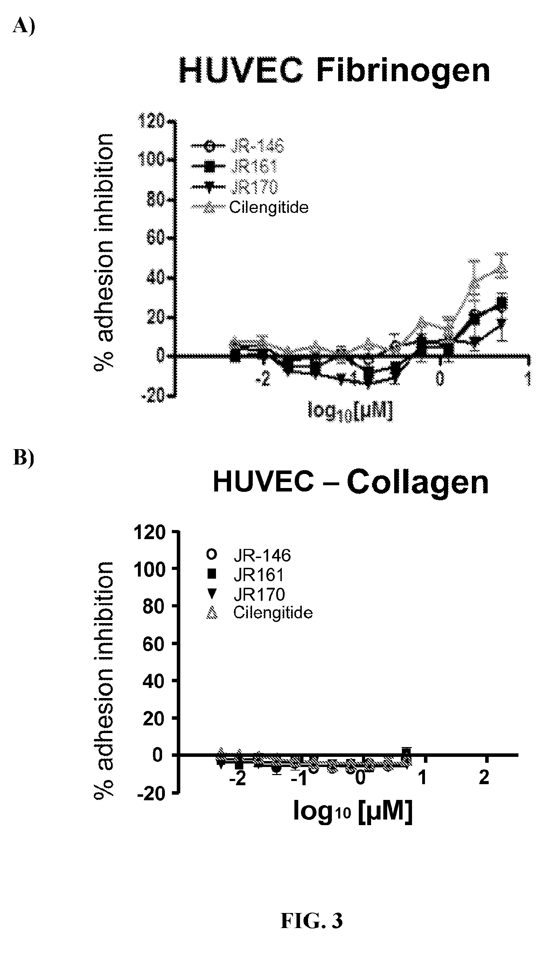

- **Figure 3.**: Cell adhesion inhibition curves for compounds 3 (JR-146), 4 (JR161) and 5 (JR170) and for cilengitide as comparative control in the HUVEC cell line. **A)** Using fibronectin (FN) as ligand. **B)** Using collagen (COL) as ligand.

### DETAILED DESCRIPTION OF THE INVENTION

The authors of the present invention have discovered that, surprisingly, the cyclic analogues of the RGD tripeptide comprising two or three thiazole rings show selective antagonist activity for αᵥβ3 over αᵥβ₅. Said antagonist activity can be identified by the methods described in the examples of the present document. Likewise, thanks to the specificity they show, the cyclic peptides of the present invention are useful for the treatment and/or diagnosis of those pathologies where the αᵥβ₃ integrin is overexpressed.

### COMPOUNDS OF THE INVENTION

Therefore, in a first aspect, the invention relates to a compound of general formula (I): 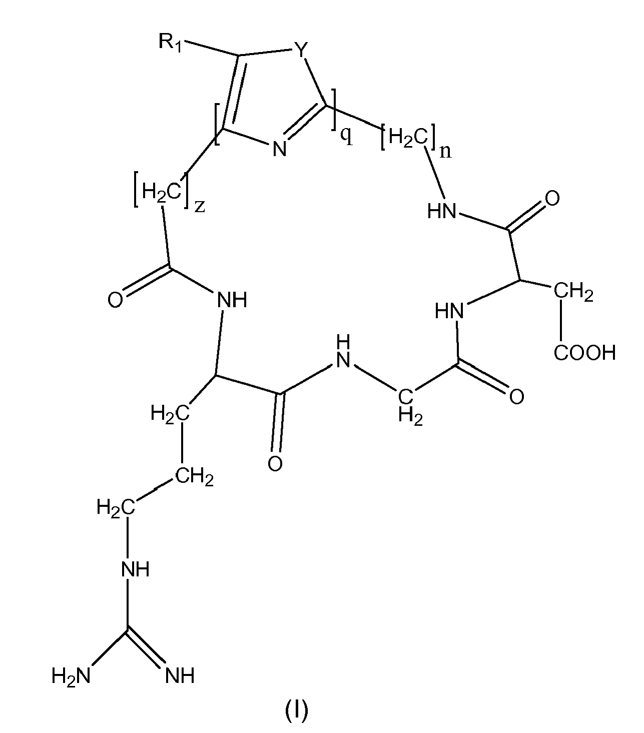 its stereoisomers, mixtures thereof and/or its pharmaceutically acceptable salts wherein:
Y is independently selected for each heterocycle of O and S;
R₁ is independently selected for each heterocycle of the group formed by H, OH, halogen, alkoxyl, aryloxyl, alkyl, aryl, acyl, amine, cycloalkyl, carboxy, alkynylalkyl and mercaptoalkyl;
n is a whole number selected from 0 and 2;
q is a whole number selected from 2 and 3;
and z is a whole number selected from 0 and 1

The compounds of general formula (I) of the invention are constituted by a RGD skeleton, which is bound to an amino acid based on two or three heterocyclic thiazole or oxazole rings to form a cyclic peptide.

In the context of the present invention "heterocycle" is understood to mean a thiazole or oxazole ring, substituted or non-substituted. The number of heterocycles in the compounds of the present invention may range from between two or three, a number indicated by q in the compound of formula (I). In a preferred embodiment of the invention q is 2. In another preferred embodiment of the invention q is 3.

The present invention contemplates the possibility that the compounds, irrespective of whether they comprise two or three heterocyclic rings, are all thiazole or all oxazole or comprise a combination of thiazole or oxazole heterocycles. The amino acids based on a single thiazole or oxazole ring are outside the scope of the present invention. In a preferred embodiment of the invention Y is O in all heterocycles. In another even more preferred embodiment Y is S in all the heterocycles.

Each one of said heterocycles may be substituted by a R₁ radical which is independently selected for each heterocycle of the group formed by H, OH, halogen, alkoxyl, aryloxyl, alkyl, aryl, acyl, amine, cycloalkyl, carboxy, alkynylalkyl and mercaptoalkyl; preferably it is selected from H, OH, alkyl, amine, cycloalkyl, carboxy, alkynylalkyl and mercaptoalkyl; more preferably from H, OH and alkyl; even more preferably from H and alkyl; and even more preferably is H. Thus, in a preferred embodiment of the invention, R₁ is H in all the heterocycles.

The term "halogen" relates to an atom of F, Cl, Br or I.

The term "alkoxyl" relates to an -OR radical wherein R is an alkyl group. This term includes, for example and by way of non-limiting example, methoxyl, ethoxyl, isopropoxyl, n-butoxyl, isobutoxyl, 3-methylbutoxyl, 2-methylbutoxyl, preferably methoxyl and ethoxyl.

The term "aryloxyl" relates to an -OR radical wherein R is an aryl group. This term includes, for example and by way of non-limiting example, phenoxyl, benzoxyl, naphthoxyl, O-(CH₂)₁₋₆-phenyl, preferably phenoxyl and benzoxyl.

The term "alkyl" relates to a radical of saturated hydrocarbonated chain, linear or branched, which has between 1 and 12, preferably between 1 and 8, more preferably between 1 and 6, even more preferably between 1 and 4, still more preferably 1, 2 or 3 carbon atoms and which is linked to the rest of the molecule by a single bond, including, for example and by way of non-limiting example, methyl, ethyl, n-propyl, isopropyl, isobutyl, n-butyl, tert-butyl, pentyl, heptyl, octyl, decyl, dodecyl, 3-methylbutyl, 2-ethylhexyl, 2-methylbutyl, 5-methylhexyl and such like.

The term "aryl" relates to an aromatic group which has between 6 and 30, preferably between 6 and 18, more preferably between 6 and 10, even more preferably 6 or 10 carbon atoms, comprising 1, 2, 3 or 4 aromatic rings, linked by a carbon-carbon bond or condensated, including, for example and by way of non-limiting example, phenyl, naphthyl, diphenyl, indenyl, phenantryl or anthranyl among others; or an aralkyl group.

The term "aralkyl group" relates to an alkyl group substituted with an aromatic group, having between 7 and 24 carbon atoms and including, for example and by way of non-limiting example, -(CH₂)₁₋₆-phenyl, -(CH₂)₁₋₆-(1-naphthyl), -(CH₂)₁₋₆-(2-naphthyl), -(CH₂)₁₋₆-CH(phenyl)₂ and such like.

The term "acyl" relates to a group derived from a carboxylic acid of formula R-CO- wherein R is an alkyl group or aryl. This term includes, for example and by way of non-limiting example, acetyl, ethanoyl, benzoyl, isopropylpentanoyl, butanoyl, preferably acetyl and ethanoyl.

The term "amine" relates to a group of formula -NH₂, -NHR or -NRR', optionally quaternized, wherein R and R' are alkyl radicals such as those previously described and independently selected. The term amine includes, for example and by way of non-limiting example, methylamine, ethylamine, methylpropylamine and such like.

The term "cycloalkyl" relates to a saturated mono- or polycyclic aliphatic group which has between 3 and 24, preferably between 3 and 16, more preferably between 3 and 14, even more preferably between 3 and 12, still more preferably 3, 4, 5 or 6 carbon atoms and which is linked to the rest of the molecule by a single bond, including, for example and by way of non-limiting example, cyclopropyl, cyclobutyl, cyclopentyl, cyclohexyl, cycloheptyl, methyl cyclohexyl, dimethyl cyclohexyl, octahydroindene, decahydronaphthalene, dodecahydrophenalene and such like.

The term "carboxy" or "carboxyl" relates to a -COOH group.

The term "alkynylalkyl" relates to an alkyl group with one or more alkynyl substituents, preferably with one alkynyl substituent. "Alkynyl" is understood to mean a group, linear or branched, which has between 2 and 24, preferably between 2 and 16, more preferably between 2 and 14, even more preferably between 2 and 12, still more preferably 2, 3, 4, 5 or 6 carbon atoms with one or more triple carbon-carbon bonds, preferably 1, 2 or 3 triple carbon-carbon bonds, conjugated or non-conjugated, which is linked to the rest of the molecule by a single bond. Examples of alkynylalkyl groups are, by way of non-limiting example, 2-propinyl, 2-pentinyl, 1-methyl-2-propinyl and such like.

The term "mercaptoalkyl" relates to an alkyl substituent which is in turn substituted by one or more mercapto groups. Non-limiting examples of mercaptoalkyl groups are 3-mercaptopropyl, 2-mercaptoethyl and such like. As understood in this technical area, there may be a certain degree of substitution on the previously defined groups. Thus, the specified radical may be substituted in one or more available positions by one or more substituents, preferably in 1, 2 or 3 positions, more preferably in 1 or 2 positions, still more preferably in 1 position. Said substituents include, for example and by way of non-limiting example, alkyl C₁-C₄; hydroxyl; alkoxyl C₁-C₄; amine; aminoalkyl C₁-C₄; carbonyloxyl C₁-C₄; oxycarbonyl C₁-C₄; halogen; cyano; nitro; azide; alkylsulfonyl C₁-C₄; thiol; alkylthio C₁-C₄; aryloxyl such as phenoxyl, aryl, acyl, cycloalkyl, carboxy, alkynylalkyl, mercaptoalkyl.

The heterocycles that constitute the amino acid based on thiazole or oxazole may be directly bound to the carboxyl group of said amino acid or through a carbon atom. Thus, in a preferred embodiment of the invention z is 0.

Furthermore, the heterocycles that constitute the amino acid based on thiazole or oxazole may be directly bound to the amine group of said amino acid or through one or two carbon atoms. In a preferred embodiment of the invention n is 2 and in an even more preferred embodiment n is 1.

The compounds of the present invention include the Arg-Gly-Asp (RGD) tripeptide.

In the present description the abbreviations used for the amino acids follow the rules of the Commission on Biochemical Nomenclature of the IUPAC IUB specified in Eur. J. Biochem., 1984, 138:9-37 and in J. Biol. Chem., 1989, 264:633-673.

In this way, for example, Gly represents NH₂-CH₂-COOH, Glyrepresents NH₂-CH₂-CO-, -Gly represents -NH-CH₂-COOH and -Glyrepresents -NH-CH₂-CO-. Therefore, the dash, which represents the peptide bond, eliminates the OH from the 1-carboxyl group of the amino acid (here conventionally represented in unionized form) when it is positioned to the right of the symbol, and eliminates the H from the 2-amino group of the amino acid when it is positioned to the left of the symbol. Both modifications can be applied to the same symbol.

In a preferred embodiment of the invention, the compound is selected from a compound of formula (II) or of formula (III), preferably it is the compound of formula (III), more preferably the compound of formula (II).

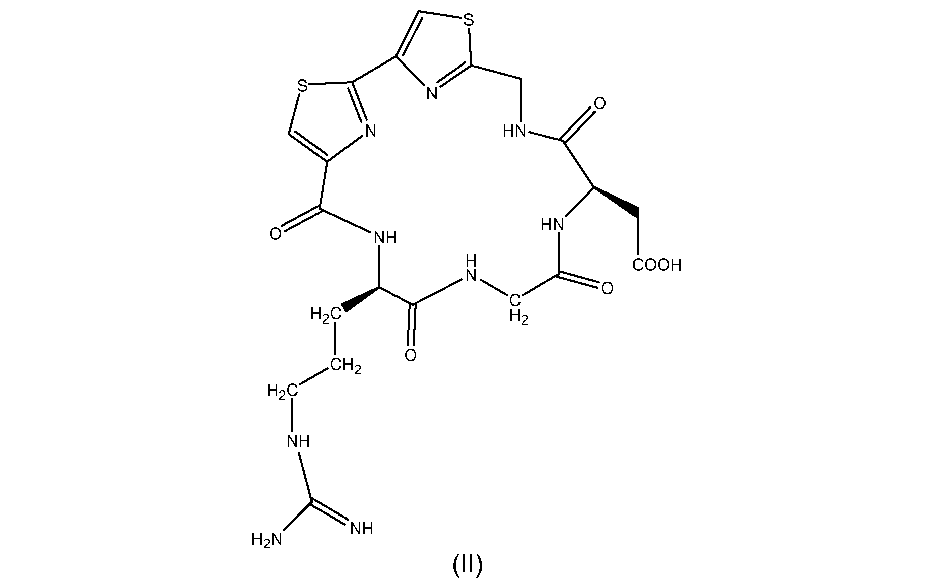

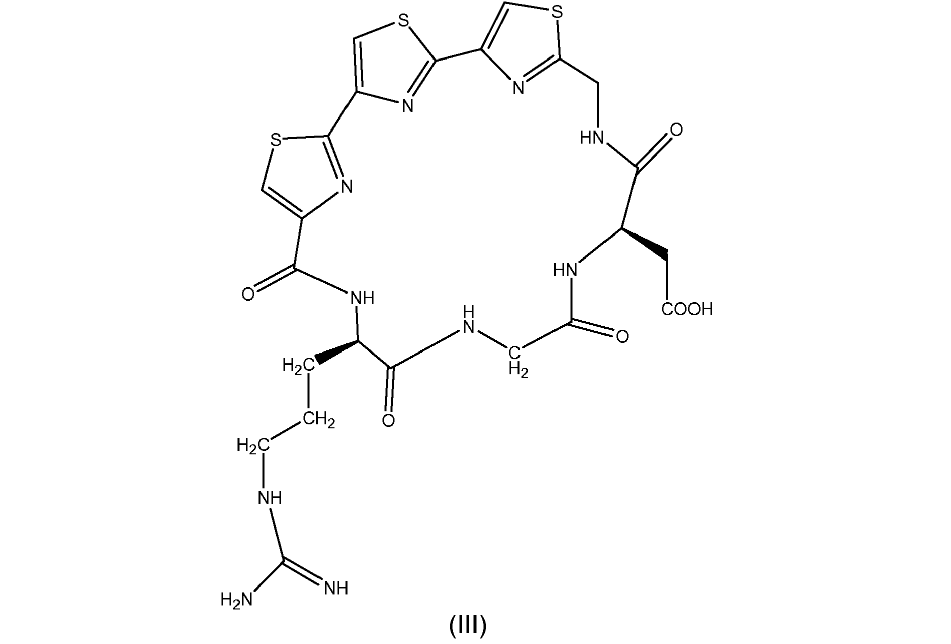

The compounds of the present invention may exist as stereoisomers or mixture of stereoisomers. The person skilled in the art understands a stereoisomer to be compounds consisting of the same atoms linked in the same sequence of bonds but which have different three-dimensional structures that are not interchangeable, such as diastereoisomers or enantiomers. For example, the amino acids that form the compounds of the present invention may have L-, D- configuration or be racemic, independently of one another. Therefore, it is possible to obtain isomeric mixtures as well as racemic mixtures or diastereomeric mixtures, or pure diastereomers or enantiomers, depending on the number of asymmetric carbons and on what isomers or isomeric mixtures are present. The preferred structures of the compounds of the invention are pure isomers, i.e. a single enantiomer or diastereomer, and preferably are L-amino acids.

When nothing is indicated, it is understood that the amino acid is L or D, or mixtures of both, racemic or non-racemic.

The scope of the present invention also includes the pharmaceutically acceptable salts of the compounds provided by this invention. The term "pharmaceutically acceptable salts", as used in the present document, relates to a salt recognized for its use in animals, and more particularly in human beings, and includes the salts used to form base addition salts, whether inorganic, such as, for example and by way of non-limiting example, lithium, sodium, potassium, calcium, magnesium, manganese, copper, zinc or aluminium among others, whether organic, such as, for example and by way of non-limiting example, ethylamine, diethylamine, ethylenediamine, ethanolamine, diethanolamine, arginine, lysine, histidine or piperazine, among others, or acid addition salts, whether organic, such as, for example and by way of non-limiting example, acetate, citrate, lactate, malonate, maleate, tartrate, fumarate, benzoate, aspartate, glutamate, succinate, oleate, trifluoroacetate, oxalate, pamoate or gluconate among others, or inorganic, such as, for example and by way of non-limiting example, chloride, sulphate, borate or carbonate among others. The nature of the salt is not critical, whenever it is pharmaceutically acceptable. The pharmaceutically acceptable salts of the peptides of the invention may be obtained by conventional methods, well known in the state of the art (Berge SM et al. J. Pharm. Sci. 1977, 66:1-19).

Also included in the scope of the present invention are the crystalline forms of the compounds of the invention such as free compounds or solvates.

The solvating techniques are well known in the state of the art. Suitable solvates are those pharmaceutically acceptable. In a particular embodiment the solvate is a hydrate.

A pharmaceutically acceptable form is a crystalline form that can be incorporated as such in a pharmaceutical composition. In the case of salts and solvates the residual solvents and the additional ionic compounds cannot be toxic.

The compounds of the invention may also have different polymorphic forms, all included in the scope of the present invention.

### PROCESS FOR OBTAINING THE COMPOUNDS OF THE INVENTION

In another aspect, the invention relates to a process for obtaining a compound of general formula (I) according to the invention, its stereoisomers, mixtures thereof, or its pharmaceutically acceptable salts comprising the cyclization between the amino-terminal end and the carboxyl-terminal end of a linear peptide selected from the group consisting of:
Arg-Gly-Asp-X
Gly-Asp-X-Arg
Asp-X-Arg-Gly and
X-Arg-Gly-Asp
wherein X is an amino acid based on thiazole and/or oxazole nuclei of formula (IV) 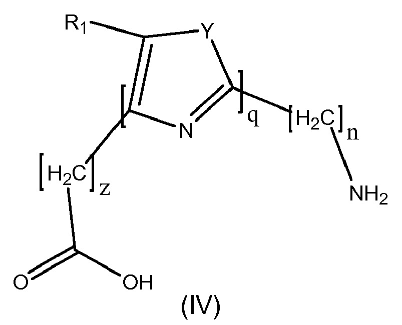 wherein:
Y is independently selected for each heterocycle of O and S;
R₁ is independently selected for each heterocycle of the group formed by H, OH, halogen, alkoxyl, aryloxyl, alkyl, aryl, acyl, amine, cycloalkyl, carboxy, alkynylalkyl and mercaptoalkyl;
n is a whole number selected from 0 and 2;
q is a whole number selected from 2 and 3;
and z is a whole number selected from 0 and 1

The synthesis or process for obtaining the compounds of the invention, their stereoisomers or their pharmaceutically acceptable salts may be performed according to conventional methods, known in the state of the art.

The linear peptide from which the cyclic compounds of the invention may be obtained is synthesized using solid phase or solution peptide synthesis methods, preferably in solid phase.

The solid phase synthesis methods are described, for example, in Stewart JM and Young JD ("Solid Phase Peptide Synthesis", 1984, 2nd edition. Pierce Chemical Company, Rockford, Illinois); Bodanzsky M and Bodanzsky A ("The practice of Peptide Synthesis", 1984, Springer Verlag, New Cork); Lloyd-Williams P, Albericio F and Giralt E ("Chemical Approaches to the Synthesis of Peptides and Proteins", 1997, CRC, Boca Raton, FL, USA).

The solution synthesis methods and combinations of the solid phase and solution synthesis methods are described in Kullmann W et al. (J Biol Chem, 1980, 255:8234-8238).

Preferably, the C-terminal end is bound to a solid support and the process is developed in solid phase and, therefore, comprises the coupling of an amino acid having the *N*-terminal end protected and the *C*-terminal end free to an amino acid having the *N*-terminal end free and the *C*-terminal bound to a polymeric support; elimination of the protective group of the *N*-terminal end; and repetition of this sequence as many times as necessary to thus obtain a tetrapeptide, followed, finally, by cleavage of the synthesized peptide from the original polymeric support.

The functional groups of the side chains of the amino acids are maintained conveniently protected with temporary or permanent protective groups throughout synthesis, and may be deprotected simultaneously or orthogonally to the peptide cleavage process from the polymeric support.

Alternatively, the solid phase synthesis may be performed by a convergent strategy coupling a peptide fragment to the polymeric support or to a peptide fragment previously bound to the polymeric support. Convergent synthesis strategies are widely known by persons skilled in the art and are described in Lloyd-Williams P et al. (Tetrahedron 1993, 49:11065-11133).

The process may comprise the additional stages of deprotection of the N-terminal and C-terminal ends and/or cleavage of the peptide from the polymeric support in indifferent order, using standard processes and conditions known in the state of the art.

In the context of the present invention "linear peptide" is understood to be a tetrapeptide defined by one the following formulas:
Arg-Gly-Asp-X
Gly-Asp-X-Arg
Asp-X-Arg-Gly
X-Arg-Gly-Asp
wherein X is an amino acid based on nuclei of thiazole or oxazole of formula (IV), so that when said linear peptide is cyclized the R-G-D sequence will be uninterrupted.

The "amino-terminal end" or "N-terminal" of the peptide is the left end of the peptide, which ends with an amino acid which has a free amine group that may or may not be protected. The "carboxyl-terminal end" or "C-terminal" of the peptide is the right end of the peptide, which finalizes with an amino acid with a free carboxyl group, which may or may not be protected.

Said linear peptides may be anchored to the polymeric support by their carboxyl-terminal end or may be in solution, once the peptide has been cleaved from the polymeric support.

Once the linear peptide has been obtained, this must be cyclized to produce the compounds of the invention.

In the context of the present invention, "cyclization" is understood to be the process whereby a ring is formed via a head-to-tail amide bond, i.e. the bond occurring between the amino-terminal end and the carboxyl-terminal end of a linear peptide according to the invention.

The method of the invention includes both the cyclization of the peptide in solution and the cyclization of the peptide in solid phase. The person skilled in the art will understand that when the synthesis of the linear peptide has been performed in solid phase, to carry out the cyclization of the peptide in solution the peptide must previously be cleaved from the polymeric support, preferably by acid treatment. In this case, after the cyclization of the peptide in solution the protective groups will be eliminated, preferably with trifluoroacetic acid. On the other hand, when the cyclization of the peptide is performed in solid phase, this may be carried out immediately after synthesis of the linear peptide in solid phase. Later, cleavage of the peptide from the polymeric support will be carried out as will simultaneous elimination of the protective groups, preferably by treatment with trifluoroacetic acid.

The cyclization of the linear peptide is carried out in the presence of a suitable solvent, such as N,N-dimethylformamide (DMF), and a base, such as, for example N,N-diisopropylethylamine (DIPEA) or triethylamine, or an additive, such as, for example 1-hydroxybenzotriazole (HOBt) or 1-hydroxyazabenzotriazole (HOAt), and a dehydrating agent, such as, for example a carbodiimide, a uronium salt, a phosphonium salt or an amidinium salt among others, to thus obtain a cyclic peptide according to the invention of general formula (I), which has the functional groups that do not participate in formation of the peptide bond conveniently protected with temporary or permanent protective groups. In a preferred embodiment, the cyclization of the linear peptide is carried out in the presence of DMF, DIPEA and a phosphonium salt.

The person skilled in the art will easily understand that the deprotection/cleavage steps of the C-terminal and *N*-terminal ends may be carried out in indistinct order, in accordance with processes known in the art (Smith MB and March J. 1999 "March's Advanced Organic Chemistry Reactions, Mechanisms and Structure", 5th edition, John Wiley & Sons, 2001).

The term "protective group", in the context of the present invention, relates to a group that blocks an organic functional group and may be eliminated in controlled conditions. The protective groups, their relative reactivities and the conditions where they remain inert are known by the person skilled in the art.

Examples of protective groups representative for the amine group are the amides, such as amide acetate, amide benzoate, amide pyvalate; carbamates, such as benzyloxycarbonyl (Cbz or Z), 2-chlorobenzyl (CIZ), *para*-nitrobenzyloxycarbonyl (pNZ), *tert*-butyloxycarbonyl (Boc), 2,2,2-trichloroethyloxycarbonyl (Troc), 2-(trimethylsilyl)ethyloxycarbonyl (Teoc), 9-fluorenylmethyloxycarbonyl (Fmoc) or allyloxycarbonyl (Alloc), trityl (Trt), methoxytrityl (Mtt), 2,4-dinitrophenyl (Dnp), *N*-[1-(4,4-dimethyl-2,6-dioxocyclohex-1-ylidene)ethyl] (Dde), 1-(4,4-dimethyl-2,6-dioxocyclohexylidene)-3-methyl-butyl (ivDde), 1-(1-adamantyl)-1-methylethoxycarbonyl (Adpoc), among others; preferably, Boc or Fmoc; more preferably Fmoc. In a preferred embodiment, the temporary protective group of the amino-terminal end of each amino acid is protected with Boc or Fmoc, more preferably with Fmoc.

Examples of protective groups representative for the carboxyl group are the esters, such as *tert*-butyl (tBu) ester, allyl (All) ester, triphenylmethyl ester (trityl ester, Trt), cyclohexyl (cHx) ester, benzyl (Bzl) ester, *ortho*-nitrobenzyl ester, *para*-nitrobenzyl ester, *para*-methoxybenzyl ester, trimethylsilylethyl ester, 2-phenylisopropyl ester, fluorenylmethyl (Fm) ester, 4-(*N*-[1-(4,4-dimethyl-2,6-dioxocyclohexylidene)-3-methylbutyl]amino)benzyl (Dmab) ester, among others; preferred protective groups of the invention are the esters of All, tBu, cHex, Bzl and Trt; more preferably tBu.

The trifunctional amino acids may be protected during the synthetic process with temporary or permanent protective groups orthogonal to the protective groups of the *N*-terminal and *C*-terminal ends. The side chain of aspartic acid may be protected with any of the protective groups of the carboxyl group previously described, preferably Bzl and tBu, more preferably tBu.

Nevertheless, the guanidine group of arginine remains protonated under the conditions given in the protection of the α groups, in the formation of the peptide bond and in the deprotection, for which reason it may not be protected. Nevertheless, it is recommendable to protect it due to the fact that it may have solubility problems. To protect the guanidine group of arginine, protective groups are usually used such as 2,2,4,6,7-pentamethyldihydrobenzofuran-5-sulfonyl (Pbf) or 2,2,5,7,8-pentamethylchroman-6-sulfonyl (Pmc), more preferably Pbf.

Examples of these and other additional protective groups, their introduction and their elimination, may be found described in the literature (Greene TW and Wuts PGM, (1999) "Protective groups in organic synthesis" John Wiley & Sons, New York; Atherton B and Sheppard RC (1989) "Solid Phase Peptide Synthesis: A practical approach" IRL Oxford University Press). The term "protective groups" also includes the polymeric supports used in the solid phase synthesis.

When the synthesis is performed totally or partially in solid phase, solid supports to be used in the process of the invention may include polystyrene supports, polyethylene glycol grafted on polystyrene and such like, such as, for example and by way of non-limiting example, *p*-methylbenzhydrylamine resins (MBHA) (Matsueda GR et al., Peptides 1981, 2:45-50), 2-chlorotrityl resins (Barlos et al. Tetrahedron Lett 1989, 30:3943-3946; Barlos K et al. Tetrahedron Lett 1989, 30:3947-3951), TentaGel^{®} resins (Rapp Polymere GmbH), ChemMatrix^{®} resins (Matrix Innovation, Inc) and such like, which may or may not include a labile spacer such as 5-(4-aminomethyl-3,5-dimethoxyphenoxy) valeric acid (PAL) (Albericio F et al. J Org Chem 1990, 55:3730-3743), 2-[4-aminomethyl-(2,4-dimethoxyphenyl)] phenoxyacetic acid (AM) (Rink H. Tetrahedron Lett 1987, 28:3787-3790), Wang (Wang SS. J Am Chem Soc 1973, 95:1328-1333) and such like which allow the cleavage of the semiprotected peptide and formation of the cycle in solution with a deprotection step in solution or the cyclization in solid phase and later deprotection and simultaneous cleavage of the peptide.

In a preferred embodiment of the invention, the cyclization is performed in solution. In another preferred embodiment of the invention, the cyclization is performed in solid phase.

In preferred embodiments of the invention, the compound prepared by this method may be any compound of those described within the context of the present invention.

In a preferred embodiment, the heterocycle found closest to the *C-*terminal group of amino acid X is directly bound to said *C*-terminal group, i.e. the value of z in the amino acid of formula (IV) is 0.

In another preferred embodiment, the heterocycle closest to the *N-*terminal group of amino acid X is bound to said *N*-terminal group through a carbon atom, i.e. the value of n in the amino acid of formula (IV) is 1.

In another preferred embodiment, the heterocycle or heterocycles that form part of the amino acid of formula (IV) are thiazoles, i.e. the value of Y in the amino acid of formula (IV) is S.

In another preferred embodiment, the heterocycle or heterocycles do not have substituents in position 5, i.e. the R₁ group is H in all the heterocycles.

In another preferred embodiment, the linear peptide undergoing a cyclization reaction is
Asp-X-Arg-Gly

In another preferred embodiment, X is an amino acid selected from the group of the amino acid of formula (V) and of the amino acid of formula (VI), preferably of the amino acid of formula (VI), more preferably of the amino acid of formula (V).

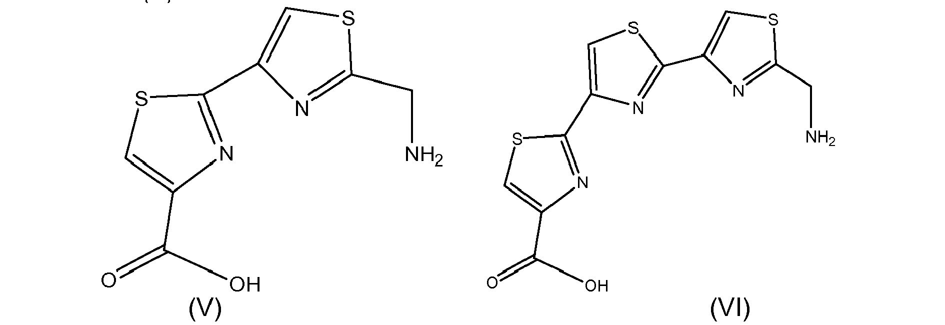

The synthesis of the amino acid X is performed by methods known in the state of the art, depending on whether the heterocycles are oxazoles or thiazoles.

The preparation of the amino acids based on thiazoles may be carried out through a stepwise route adapting the methods described in the literature (Koerber-Pie K and Massiot G. J Het Chem 1995, 32:1309-1315).

The preparation of the amino acids based on oxazoles may be carried out using analogous techniques including coupling processes of organometallic derivatives and cyclodehydration, as described in Tera M (Tera M et al. Angew. Chem. Int. Ed. 2008, 47:5557-5560) and literature cited therein.

The reaction products may be purified, if necessary, by conventional methods such as crystallization or chromatography.

When the processes described above to obtain the compounds of the invention produce mixtures of stereoisomers, they may be used as such or separated using conventional methods, such as preparative chromatography. In the case that there are stereocentres present, compounds in racemic form may be prepared. Alternatively, the individual enantiomers may be prepared by enantioselective synthesis or by resolution.

The invention is also aimed at the synthesis intermediates, i.e. linear peptides that are used later for the cyclization.

In an additional aspect, the invention also relates to a linear peptide selected from one of the following formulas:
Arg-Gly-Asp-X
Gly-Asp-X-Arg
Asp-X-Arg-Gly and
X-Arg-Gly-Asp
wherein X is an amino acid based on thiazole and/or oxazole nuclei of formula (IV) 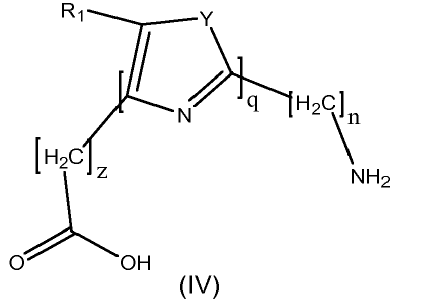 wherein:
Y is independently selected for each heterocycle of O and S;
R₁ is independently selected for each heterocycle of the group formed by H, OH, halogen, alkoxyl, aryloxyl, alkyl, aryl, acyl, amine, cycloalkyl, carboxy, alkynylalkyl and mercaptoalkyl;
n is a whole number selected from 0 and 2;
q is a whole number selected from 2 and 3;
and z is a whole number selected from 0 and 1
and wherein the residues of Arg and Asp are optionally protected.

In a preferred embodiment of the invention the linear peptides of the invention are protected using any protective group known in the state of the art or, preferably, using the protective groups mentioned above for each one of the active groups present in said amino acid. In another preferred embodiment the linear peptides of the invention are deprotected.

### PHARMACEUTICAL COMPOSITIONS OF THE INVENTION

Another aspect of the invention is a pharmaceutical composition comprising a pharmaceutically effective quantity of at least one compound of general formula (I), its stereoisomers, mixtures thereof and/or its pharmaceutically acceptable salts, and at least one pharmaceutically acceptable excipient.

As used in the present invention, the expression "pharmaceutical composition" relates to a formulation which has been adapted to administer a predetermined dose of one or several therapeutically useful agents to a cell, a group of cells, an organ, a tissue or an animal where there is an overexpression of the αᵥβ₃ integrin.

The pharmaceutical compositions containing the compounds of the invention, their stereoisomers, mixtures thereof and/or their pharmaceutically acceptable salts may be administered by any appropriate route, such as oral, topical or parenteral route for which it will include the pharmaceutically acceptable excipients necessary for the formulation of the form of administration desired. The preferred administration route of the pharmaceutical composition is endovenous route.

The compositions of the invention are suitable for administration to any type of mammal, preferably a human being.

"Pharmaceutically effective quantity" relates to a quantity capable of exercising a therapeutic effect, and which may be determined by the person skilled in the art using typically used means.

The compositions of the invention may contain one or more compounds according to the invention, its stereoisomers, mixtures thereof and/or its pharmaceutically acceptable salts.

The compositions of the invention may also contain one or several additional compounds for the treatment of a pathology associated to an undesired cellular proliferation, or of a pathology associated to an undesired angiogenesis or for the treatment of metastasis.

The pharmaceutical compositions are prepared by conventional means with pharmaceutically acceptable excipients.

"Pharmaceutically acceptable excipient" is understood to be an inactive substance therapeutically speaking, used to incorporate the active principle and which is acceptable for the patient from a pharmacological/toxicological viewpoint and for the pharmaceutical chemist that manufactured it from a physical/chemical standpoint with respect to the composition, formulation, stability, acceptance by the patient and bioavailability.

The number and the nature of the pharmaceutically acceptable excipients depend on the desired administration form. The pharmaceutically acceptable excipients are known by the person skilled in the art (Faulí and Trillo C. (1993) "Tratado de Farmacia Galénica", Luzán 5, S.A. Ediciones, Madrid). Said compositions may be prepared by the conventional methods known in the state of the art ("Remington: The Science and Practice of Pharmacy", 20th edition (2003) Genaro A.R., ed., Lippincott Williams & Wilkins, Philadelphia, US).

### THERAPEUTIC USES OF THE COMPOUNDS OF THE INVENTION

The compounds of the invention, due to their selective antagonist activity of the αᵥβ₃ integrin, are applicable in those diseases where said integrin is overexpressed. Therefore, given the role that the integrins play in tumour progression and in angiogenesis, the peptides may be used in therapy. Thus, in another aspect, the invention relates to a compound according to the invention for use in medicine.

Specifically, the αᵥβ₃ integrin is expressed on the surface or a large variety of cells, including osteoclasts, vascular smooth muscle cells, endothelial cells and several tumour cells, and it is involved in numerous biological processes such as adhesion of osteoclasts to the bone, migration of vascular smooth muscle cells and angiogenesis. As a result, the ligands of the αᵥβ₃ integrin are candidate drugs for cancer treatment (Arndt T et al. 2004 in: Weber GF (ed) Cancer Therapy: Molecular Tumor. Horizon Bioscience, Wymondham, pages 93-141; Haubner R et al. 1997 Angew Chem Int Ed 36:1375; Yun Z et al. 1996 Cancer Res 56:3103), osteoporosis (Schaffner P and Dard MM. 2003 Cell Mol Life Sci 60:119; Keenan RM et al. 1997 J Med Chem 40:2289), proliferative diabetic retinopathy (Friedlander M et al. 1996 Proc Natl Acad Sci USA 93:9764), restenosis (Matsuno H et al. 1994 Circulation 90:2203; Choi ET et al. 1994 J Vasc Surg 19:125) and acute renal insufficiency (Noiri E et al. 1994 Kidney Int 46:1050; Romanov V et al. 1997 Kidney Int 52:93; Goligorsky MS et al. 1997 Kidney Int 51:1487).

Furthermore, since the αᵥβ₃ integrin is overexpressed in the endothelium of the tumour tissue, it may contribute to malignity favouring angiogenesis. Angiogenesis is a process dependent on anchoring for which purpose it is necessary the interaction between vascular integrins and extracellular matrix proteins (Brooks PC et al. Science 1994, 264:569-571). Therefore, said process may be inhibited by interfering between the binding of the endothelial cells to the extracellular matrix (Strömblad S and Cheresh DA. 1996 Chem Biol 3:881; Strömblad S and Cheresh DA. 1996 Trends Cell Biol 6:462). The inhibition of the interaction between the cell and the matrix mediated by αᵥβ₃ leads to the apoptosis of the activated endothelial cells and suppresses the formation of blood vessels. It is known that cyclic peptides are capable of inhibiting said interaction and, therefore, may inhibit tumour-induced angiogenesis (Haubner R et al. 1997 Angew Chem Int Ed 36:1375; Brooks PC et al. 1994 Cell 79:1157) and may be used in the treatment of cancer, as exemplified by the compound Cilengitide®, which is currently in clinical phase III for the treatment of glioblastoma (brain tumour).

Thus, in one aspect, the invention relates to the use of a compound according to the invention for the preparation of a medicament for the prevention and/or the treatment of a disease associated to an undesired cellular proliferation, a disease associated to an undesired angiogenesis, metastasis, restenosis, osteoporosis, an inflammatory disease, proliferative diabetic retinopathy and/or acute renal insufficiency.

In another aspect, the invention relates to a compound according to the invention for use in the preparation of a medicament for the prevention and/or the treatment of a disease associated to an undesired cellular proliferation, a disease associated to an undesired angiogenesis, metastasis, restenosis, osteoporosis, an inflammatory disease, proliferative diabetic retinopathy and/or acute renal insufficiency.

In another aspect, the invention relates to a method of treatment or the prevention in an individual of a disease associated to an undesired cellular proliferation, a disease associated to an undesired angiogenesis, metastasis, restenosis, osteoporosis, an inflammatory disease, proliferative diabetic retinopathy and/or acute renal insufficiency comprising the administration to said subject of a compound according to the invention.

"Medicament" is understood to be a pharmaceutical composition comprising one of the compounds of the invention or a pharmaceutically acceptable salt thereof. The preparation of a medicament from the compounds of the present invention may also include the derivatization of said compounds to form prodrugs, which are then transformed "in vivo" in the compounds of the invention. Said derivatized compounds are evident for a person skilled in the art and include, depending on the functional groups of the molecule and without limitation, esters, amino acid esters, phosphate esters and sulphonate esters of metal salts, carbamates and amides. Particularly preferred are those derivatives or prodrugs that increase the bioavailability of the compounds of the invention when said compounds are administered to the patient or that improve administration of the original compound due to their biological behaviour.

"Prevention" is understood to mean the administration of a compound according to the invention or of a medicament containing it or of its pharmaceutically acceptable salts in an initial or early stage of the disease, or also to avoid its appearance.

The term "treatment" is used to designate the administration of a compound according to the invention or of a medicament containing it or of its pharmaceutically acceptable salts to control the progression of the disease before or after clinical signs have appeared. Control of the disease progression is understood to mean the beneficial or desired clinical results that include, but are not limited to, reduction of the symptoms, reduction of the duration of the disease, stabilization of pathological states (specifically to avoid additional deterioration), delaying the progression of the disease, improving the pathological state and remission (both partial and total). The control of progression of the disease also involves an extension of survival, compared with the expected survival if treatment was not applied.

In the context of the present invention, a "disease associated to an undesired cellular proliferation" includes the growth, progression and metastasis of cancer and tumours. Examples of diseases associated to an undesired cellular proliferation and which may be treated in accordance with the methods described in the present invention are cancer, restenosis, arteriosclerosis, angiogenic diseases, fibrosis, dermatological diseases and inflammatory diseases.

In a particular embodiment of the invention, the disease associated to an undesirable cellular proliferation is cancer.

The terms "cancer" and "tumour" refer to the physiological condition in mammals characterized by unregulated cell growth. The compounds of the present invention are useful in the treatment of breast, heart, lung, small intestine, colon, spleen, kidney, bladder, head, neck, ovarian, prostate, brain, pancreas, skin, bone, bone marrow, blood, thymus, uterus, testicles and liver tumours. In particular, tumours which may be treated with compounds of the invention include adenoma, angiosarcoma, astrocytoma, epithelial carcinoma, germinoma, glioblastoma, glioma, hemangioendothelioma, hemangiosarcoma, hematoma, hepatoblastoma, leukaemia, lymphoma, medulloblastoma, melanoma, neuroblastoma, osteosarcoma, retinoblastoma, rhabdomyosarcoma, sarcoma, and teratoma. In particular, the tumour/cancer is selected from the group of acral lentiginous melanoma, actinic keratosis adenocarcinoma, adenoid cystic carcinoma, adenomas, adenosarcoma, adenosquamous carcinoma, astrocytic tumours, bartholin gland carcinoma, basal cell carcinoma, bronchial gland carcinoma, capillary carcinoid, carcinoma, carcinosarcoma, cholangiocarcinoma, cystadenoma, endodermal sinus tumour, endometrial hyperplasia, endometrial stromal sarcoma, endometrioid adenocarcinoma, ependymal sarcoma, Swing's sarcoma, focal nodular hyperplasia, germ cell tumours, glioblastoma, glucagonoma, hemangioblastoma, hemangioendothelioma, hemangioma, hepatic adenoma, hepatic adenomatosis, hepatocellular carcinoma, insulinoma, intraepithelial neoplasia, interepithelial squamous cell neoplasia, invasive squamous cell carcinoma, large cell carcinoma, leiomyosarcoma, melanoma, malignant melanoma, malignant mesothelial tumour, medulloblastoma, medulloepithelioma, mucoepidermoid carcinoma, neuroblastoma, neuroepithelial adenocarcinoma, nodular melanoma, osteosarcoma, papillary serous adenocarcinoma, pituitary tumours, plasmacytoma, pseudosarcoma, pulmonary blastoma, renal cell carcinoma, retinoblastoma, rhabdomyosarcoma, sarcoma, serous carcinoma, small cell carcinoma, soft tissue carcinoma, somatostatin-secreting tumour, squamous carcinoma, squamous cell carcinoma, undifferentiated carcinoma, uveal melanoma, verrucous carcinoma, vipoma, Wilm's tumour. Even more preferably, the tumour/cancer to be treated with the compounds of the invention include intracerebral cancer, head and neck cancer, rectal cancer, astrocytoma, preferably a grade II, III or IV astrocytoma, glioblastoma, preferably glioblastoma multiforme, small cell cancer, and non-small cell cancer, preferably non-small cell lung cancer, metastatic melanoma, androgen-independent metastatic prostate cancer, androgen-dependent metastatic prostate cancer and breast cancer. In a preferred embodiment the tumour that may be treated with the compounds of the invention is glioblastoma.

In the context of the present invention, "angiogenesis" is understood to mean the physiological process that consists of the formation of new blood vessels from existing blood vessels. Angiogenesis is also known as neovascularization.

The expression "diseases associated to an undesired angiogenesis" relates to all those diseases where pathogenic angiogenesis occur i.e. when said process is harmful or undesirable, whether cancerous or not. The scope of the present invention thus excludes the treatment of angiogenesis in situations where it is necessary, such as wound healing. Diseases associated to an undesired angiogenesis which may be treated with the compounds in accordance with the present invention, without limitation, are inflammatory diseases, especially chronic inflammatory diseases such as rheumatoid arthritis, psoriasis, sarcoidosis and such like; autoimmune diseases; viral diseases; genetic diseases; allergic diseases; bacterial diseases; ophthalmological diseases such as diabetic retinopathy, premature retinopathy, proliferative atrial retinopathy, retinal vein oclusion, macular degeneration, senile discoid macular degeneration, neovascular ocular glaucoma, choroidal neovascularization diseases, retinal neovascularization diseases, rubeosis (angle neovascularization), corneal graft rejection, retrolental fibroplasia, epidermal keratoconjunctivitis, vitamin A deficiency, contact lens exhaustion, atopical keratitis, superior limbic keratitis, pterygium dry eye, Sjögrens syndrome, acne rosacea, phlyctenulosis, syphilis, micobacterial infections, lipid degeneration, burns with corrosive substances, bacterial ulcers, mycotic ulcers, protozoan infections, Kaposi sarcoma, Mooren's ulcer, Terrien marginal degeneration, marginal keratolysis, scleritis, chronic retinal detachment and such like; atherosclerosis; endometriosis; obesity; cardiac insufficiency; advanced renal insufficiency; endotoxemia; toxic shock syndrome; meningitis; silicon-induced fibrosis; asbestos-induced fibrosis; apoplexia; periodontitis; gingivitis; macrocytic anaemia; refractory anaemia; 5q deletion syndrome; conditions where the vascularization is altered as infection by HIV, hepatitis, hemorrhagic telangiectasia or Rendu-Osler-Weber's disease.

In a preferred embodiment, the disease associated to an undesired angiogenesis is a disease selected from cancer, rheumatoid arthritis, psoriasis, sarcoidosis, diabetic retinopathy, premature retinopathy, retinal vein oclusion, senile discoid macular degeneration, atherosclerosis, endometriosis and obesity, preferably cancer.

In a particular embodiment the diseases associated to an undesired angiogenesis are inflammatory diseases. "Inflammatory disease" is understood to be any disease where there is an excessive or altered inflammatory response that leads to inflammatory symptoms. Said inflammatory diseases which may be treated by compounds of the invention include, without limitation, Addison's disease, acne vulgaris, alopecia areata, amyloidosis, ankylosing spondylitis, ulcerations, aphthous stomatitis, arthritis, arteriosclerosis, osteoarthritis, rheumatoid arthritis, bronchial asthma, Bechet's disease, Boeck's disease, intestinal inflammatory disease, Crohn's disease, choroiditis, ulcerative colitis, celiac's disease, cryoglobulinemia, macular degeneration, dermatitis, dermatitis herpetiformis, dermatomyositis, insulin dependent diabetes, juvenile diabetes, inflammatory demyelinating disease, Dupuytren contracture, encephalomyelitis, allergic encephalomyelitis, endophthalmia, allergic enteritis, autoimmune enteropathy syndrome, erythema nodosum leprosum, ankylosing spondylitis, idiopathic facial paralysis, chronic fatigue syndrome, rheumatic fever, cystic fibrosis, gingivitis, glomerulonephritis, Goodpasture syndrome, Graves syndrome, Hashimoto's disease, chronic hepatitis, histiocytosis, regional ileitis, iritis, disseminated lupus erythematous, systemic lupus erythematous, cutaneous lupus erythematous, lymphogranuloma, infectious mononucleosis, miastenia gravis, transverse myelitis, primary idiopathic myxedema, nephrosis, obesity, sympathetic ophthalmia, granulomatous orchitis, pancreatitis, panniculitis, pemphigus vulgaris, periodontitis, polyarteritis nodosa, chronic polyarthritis, polymyositis, acute polyradiculitis, psoriasis, chronic obstructive pulmonary disease, purpura, gangrenous pioderma, Reiter's syndrome, diabetic retinopathy, rosacea, sarcoidosis, ataxic sclerosis, progressive systemic sclerosis, scleritis, sclerodermia, multiple sclerosis, disseminated sclerosis, acute anterior uveitis, vitiligo, Whipple's disease, diseases associated to AIDS, severe combined immunodeficiency and Epstein Barr's virus such as Sjögren's syndrome, osteoarticular tuberculosis and parasitic diseases such as leishmaniasis. Preferred inflammatory diseases are rheumatoid arthritis, psoriasis, sarcoidosis, diabetic retinopathy, macular degeneration, arteriosclerosis and obesity.

In the context of the present invention, the term "metastasis" relates to the remote propagation, fundamentally by lymphatic or blood channels, of the cancer causing cells, and the growth of new tumours in the place of destination of said metastasis.

The term "restenosis" relates to the reproduction of the narrowing of an artery that certain patients undergo after having undergone an angioplasty process or stent implantation. In a preferred embodiment, the restenosis is secondary to percutaneous transluminal coronary angioplasty.

"Osteoporosis" is defined as a bone disease wherein the quantity of minerals in the bone decreases, losing strength in the part of the trabecular bone and reducing the cortical area by a defect in calcium absorption, which makes the bones brittle and susceptible to fractures and microfractures.

The term "proliferative diabetic retinopathy" makes reference to the advanced phase of the disorder known as diabetic retinopathy. Diabetic retinopathy appears as a complication of diabetes and is caused by a deterioration in the blood vessels that irrigate the retina due to high glucose levels. In the advanced phase of the disease angiogenesis and bursting of the new capillaries formed occur, which causes cloudy and defective vision, and may occasionally lead to development of scar tissue, retinal detachment and blindness.

"Acute renal insufficiency" is understood to mean the sudden loss in the capacity of the kidneys to eliminate wastes and concentrate the urine without losing electrolytes, which may be caused by a great variety of disorders.

In a preferred embodiment, the invention relates to the use of a compound according to the invention for the preparation of a medicament for the prevention and/or the treatment of cancer, diseases associated to an undesired angiogenesis and metastasis.

In an embodiment of the present invention the medicament comprises one or more compounds according to the invention as sole therapeutic agent. Nevertheless, the medicament of the invention may also contain one or several additional compounds for the treatment of diseases associated to an undesired cellular proliferation or of diseases associated to an undesired angiogenesis. Therefore, in another embodiment of the present invention, the medicament is prepared for the combined administration of a compound according to the invention and one or more therapeutic agents useful in the treatment of said diseases.

The term "therapeutic agent useful in the treatment of said diseases" relates to an agent suitable for being used in the treatment of a disease associated to an undesired cellular proliferation or of a disease associated to an angiogenesis. Cytotoxic agents that may be used in combination with the compounds of the invention for the treatment of diseases associated to an undesired cellular proliferation include, without limitation, anthracycline antibiotics such as doxorubicin and daunorubicin, taxans such as Taxol™ and docetaxel, vinca alkaloids such as vincristin and vinblastine, 5-fluorouracyl (5-FU), leucovorin, irinotecan, idarubicin, mitomycin C, oxaliplatin, raltitrexed, tamoxifen, cisplatin, carboplatin, methotrexate, actinomycin D, mitoxantrone, blenoxane or mithramycin. Anti-angiogenic agents which may be used in combination with the compounds of the invention for the treatment of diseases associated to an undesired angiogenesis include, without limitation, an anti-angiogenic agent selected from the group of paclitaxel, 2-methoxyestradiol, prinomastat, batimastat, BAY 12-9566, carboxyamidotriazole, CC-1088, dextromethorphan acetic acid, dimethylxanthenone acetic acid, endostatin, IM-862, marimastat, penicillamine, PTK787/ZK 222584, RPI.4610, squalamine lactate, SU5416, thalidomide, combretastatin, tamoxifen, COL-3, neovastat, BMS-275291, SU6668, anti-VEGF antibodies, Medi-522 (Vitaxin II), CAI, Interleukin 12, IM862, amiloride, angiostatin, angiostatin KI-3, angiostatin KI-5, captopril, DL-alpha-difluoromethylornithine, DL-alpha-difluoromethylornithine HCl, endostatin, fumagillin, herbimycin A, 4-hydroxyphenylretinamide, juglone, laminin, laminin hexapeptide, laminin pentapeptide, lavendustin A, medroxyprogesterone, minocycline, placental ribonuclease inhibitor, suramin, thrombospondin, antibodies targeted against proangiogenic factors (for example, Avastin, Erbitux, Vectibix, Herceptin); low molecular weight tryrosine kinases inhibitors of proangiogenic growth factors (for example Tarceva, Nexavar, Sutent, Iressa); mTOR inhibitors (for example Torisel); alpha, beta and gamma interferon, IL-12, matrix metalloproteinase inhibitors (for example, COL3, Marimastat, Batimastat); ZD6474, SUI248, vitaxin; PDGFR inhibitors (for example Gleevec); NM3 and 2- ME2; cyclic peptides such as cilengitide. In a particular embodiment the anti-angiogenic agent with which the compounds of the invention are combined is cilengitide.

"Combined administration" is understood to mean that the compound according to the invention may be administered jointly or separately, simultaneously, at the same time or sequentially with a therapeutic agent useful in the treatment of the pathologies previously mentioned in any order. For example, the administration of the compound of the invention may be performed first, followed by the administration of one or more therapeutic agents useful in the treatment of said pathologies; or the administration of the compound of the invention may be performed at the end, preceded by the administration of one or more therapeutic agents useful in the treatment of said pathologies; or the administration of the compound of the invention may be performed at the same time as the administration of one or more therapeutic agents useful in the treatment of said pathologies.

The person skilled in the art shall understand, in the context of the present invention, that the medicament for the combined administration of a compound according to the invention and an additional therapeutic agent useful in the treatment of said diseases may be prepared as a single dosing form or in separate dosing forms.

### DIAGNOSTIC USES OF THE COMPOUNDS OF THE INVENTION

Since αᵥβ₃ integrins are overexpressed both in tumour and endothelial cells, the αᵥβ₃ ligands, radioactively labelled, are promising diagnostic imaging agents that allow a non-invasive viewing of tumours and the monitoring of cancer therapy (Haubner R, Wester HJ, Reuning U, Senekowitsch-Schmidtke R, Diefenbach B, Kessler H, Stöcklin G, Schwaiger M (1999) J. Nucl. Med. 40:1061; Haubner R, Wester HR (2004) Curr. Pharm. Des. 10:1439).

Therefore, another aspect of the invention is related to the use of a compound according to the invention for the preparation of a diagnostic agent.

In another aspect, the invention relates to a conjugate comprising a compound in accordance with the invention and a diagnostic imaging agent.

In another aspect, the invention relates to the use of a conjugate comprising a compound in accordance with the invention and a diagnostic imaging agent for the preparation of a diagnostic agent.

In another aspect, the invention relates to a diagnostic method for the detection of a tumour that overexpresses αᵥβ₃ integrins in a patient comprising the administration to said patient of a conjugate comprising a compound in accordance with the invention and a diagnostic imaging agent and the detection of the tumour in said patient by viewing the diagnostic imaging agent.

In the context of the present invention "diagnostic agent", is understood to be an agent comprising a compound according to the invention modified so that it may be detected after being administered to an individual. Due to the affinity that the compounds of the invention show for the αᵥβ₃ integrin, the administration of the compound to a subject makes it possible to precisely detect those tissues where there is an anomalous expression of said integrin. The diagnostic agent includes, in addition to the compound of the invention, other components or excipients necessary for their use or administration.

There are several forms of introducing the diagnostic imaging agent in the cyclic peptides of the invention. In particular, this is performed via the R₁ group of the thiazole or oxazole rings (Butler JD et al. 2010. Chem. Eur. J., in press DOI: 10.1002/chem.201001492). For the conjugation of the compound of the invention with another molecule, any R₁ group suitable for this purpose can be used. Preferably, the most suitable R₁ functional groups are amine, carboxy, alkynylalkyl and mercaptoalkyl.

The diagnostic agent of the invention may be used both in *in vivo* and in *in vitro* diagnostic methods.

The *in vivo* diagnostic methods are imaging diagnostic methods. The imaging diagnostic methods that may be used with the compounds of the invention include single photon emission computed tomography (SPECT) and positron emission tomography (PET). SPECT uses the main y emitters, such as 123I, ^{99m}Tc and ¹¹¹In_{.} The most frequent positron emitters are ¹⁸ F, ¹²⁴I and ¹¹C.

"Diagnosis" is understood to mean the detection of the presence of tissues with overexpression of the αᵥβ₃ integrin that makes it possible to characterize the aggressiveness of a malignant tumour in an individual patient. It is also useful to document the expression of αᵥβ₃ in the tumours before administration of selective αᵥβ₃ drugs to enable an appropriate selection of patients and determination of the optimum dose.

The terms "diagnostic imaging agent" and "contrast agent" are used here interchangeably and refer to a biocompatible compound whose use facilitates the differentiation of different parts of the image increasing the "contrast" between those different regions of the image. The term "contrast agents" thus includes agents used to increase the quality of an image that may be generated however in the absence of said agent (as is the case, for example, in MR), as well as agents that are prerequisites for image generation (as is the case, for example, of gammagraphy). The suitable contrast agents include, without limitation, contrast agents for gammagraphy, for computed tomography, for Raman spectroscopy, for magnetic resonance imaging diagnosis (MR) and for optical imaging diagnosis.

The contrast agents for gammagraphy include radiopharmaceuticals that are normally marked with positron emitters such as ¹¹C, ¹³N, ¹⁵O, ¹⁸F, ⁸²Rb, ⁶²Cu and ⁶⁸Ga. SPECT radiopharmaceuticals are normally marked with positron emitters such as ⁹⁴mTc, ²⁰¹TI and ⁶⁷Ga. The categories of gammagraphy (positron emission tomography (PET), single photon emission computed tomography (SPECT)) are imaging diagnostic techniques of cross-sections which map the location and concentration of radiotracers marked with radionuclides. PET and SPECT may be used to locate and characterize a radionuclide measuring the metabolic activity. PET and SPECT provide information related to cell level information such as cell viability. In PET, a patient ingests or is injected with a slightly radioactive substance that emits positrons, which it can follow as the substance moves through the body. In a common application, for example, the patients are given glucose with attached positron emitters and their brains are controlled as they perform different tasks. Since the brain uses glucose when it functions, a PET image shows where the brain activity is high. In certain embodiments of the invention, a cell is marked *ex vivo* for *in vivo* PET or SPECT imaging diagnosis. Highly related with PET is single photon emission computed tomography or SPECT. The main difference between the two is that instead of a positron emitting substance, SPECT uses a radioactive tracer that emits low energy photons.

The contrast agents for CT images include, for example, iodized or brominated contrast means. Examples of these agents include iothalamate, iohexol, diatrizoate, iopamidol, ethiodol and iopanoate. Gadolinium agents have also been reported to be of use as a CT contrast agent (see, e.g. Henson et al., 2004). For example, gadopentate agents have been used as a CT contrast agent (discussed in Strunk and Schild, 2004). Computed tomography (CT) is contemplated as a type of imaging diagnosis in the context of the present invention. By taking a series of X-rays, sometimes more than a thousand, from various angles and then combining them with a computer, CT makes it possible to build up a three-dimensional image of any part of the body. A computer is programmed to display two-dimensional sections from any angle and at any depth. In CT, the intravenous injection of a radiopaque contrast agent such as those described here, can assist in identifying and delineating soft tissue masses when initial CT scans are not diagnostic.

The contrast agents for optical images include, for example, fluorescein, a fluorescein derivative, indocyanine green, Oregon green, an Oregon green derivative, rhodamine green, a rhodamine green derivative, an eosin, an erythrosin, Texas red, a Texas red derivative, malachite green, nanogold sulfosuccinimidyl ester, cascade blue, a coumarin derivative, a naphthalene, a pyridyloxazole derivative, cascade yellow dye, dapoxyl dye and several other fluorescent compounds disclosed here.

The contrast agents for magnetic resonance include, without limitation, complexes of metals selected from the group consisting of chromium (III), manganese (II), iron (III), iron (II), cobalt (II), nickel (II), copper (II), neodymium (III), samarium (III), yterbium (III), gadolinium (III), vanadium (II), terbium (III), dysprosium (III), holmium (III) and erbium (III).

The invention does not exclude the *"in vitro*" diagnostic methods wherein a diagnostic agent is used on a sample isolated from the patient. In a preferred embodiment of the invention the diagnostic agent is used for *"in vitro*" diagnosis.

Another aspect of the invention is a method for the diagnosis of the cancer comprising contacting a sample of the patient with a diagnostic agent and detecting the formation of a complex.

Another aspect of the invention relates to a compound according to the invention for use in diagnosis.

### USES OF THE COMPOUNDS OF THE INVENTION FOR THE TARGETING OF DRUGS TO TUMOURS

Given that the compounds of the invention have a high affinity for integrins of type αᵥβ₃ and that these integrins are overexpressed in tumour cells (Nasongkla N, Shuai X, Ai H, Weinberg BD, Pink J, Boothman DA, Gao JM (2004) Angew Chem Int Ed 43:6323), the compounds of the invention are suitable agents to carry compounds with cytotoxic activity to tumours. Thus, in another aspect, the invention relates to a conjugate comprising a compound in accordance with the invention and a cytotoxic agent.

In another aspect, the invention relates to the use of a conjugate comprising a compound of the invention and a cytotoxic agent or an anti-angiogenic agent or a nanocarrier for the preparation of a medicament for the treatment or the prevention of a disease associated to an undesired cellular proliferation or to an undesired angiogenesis.

In another aspect, the invention relates to a conjugate comprising a compound of the invention and a cytotoxic agent or an anti-angiogenic agent or a nanocarrier for use in the preparation of a medicament for the treatment or the prevention of a disease associated to an undesired cellular proliferation or to an undesired angiogenesis.

In another aspect, the invention relates to a method of treatment or the prevention of a disease associated to an undesired cellular proliferation or to an undesired angiogenesis in a subject comprising the administration to said subject of a pharmaceutically effective quantity of a conjugate comprising a compound of the invention and a cytotoxic agent or an anti-angiogenic agent or a nanocarrier.

The term "cytotoxic agent", as used in the present invention, relates to an agent that is capable of promoting cell death and which has the capacity for reducing growth, stopping growth or destroying cells and, in particular, cells that proliferate quickly and, even more in particular, tumour cells. Cell death may be caused by any mechanism such as, for example, apoptosis, although it is not limited to this cause, by inhibition of metabolism, interference with the cytoskeleton organization or the chemical modification of DNA. The term cytotoxic agent comprises any chemotherapeutic agent including small sized organic molecules, peptides, oligonucleotides and such like as well as radiotherapy agents such as ¹³¹I and beta particle emitters such as ⁹⁰Y.

Cytotoxic agents that may be used in the context of the present invention, include without limitation, anthracycline antibiotics such as doxorubicin and daunorubicin, taxans such as Taxol™ and docetaxel, vinca alkaloids such as vincristin and vinblastine, 5-fluorouracyl (5-FU), leucovorin, irinotecan, idarubicin, mitomycin C, oxaliplatin, raltitrexed, tamoxifen, cisplatin, carboplatin, methotrexate, actinomycin D, mitoxantrone, blenoxane, or mithramycin.

In another aspect, the invention relates to a conjugate comprising a compound in accordance with the invention and an anti-angiogenic agent.

In the case that the compounds of the invention are used in the form of conjugates with anti-angiogenic agents for the treatment of diseases associated to an undesired angiogenesis, the conjugates of the invention may comprise, by way of non-limiting illustration an anti-angiogenic agent selected from the group of paclitaxel, 2-methoxyestradiol, prinomastat, batimastat, BAY 12-9566, carboxyamidotriazole, CC-1088, dextromethorphan acetic acid, dimethylxanthenone acetic acid, endostatin, IM- 862, marimastat, penicillamine, PTK787/ZK 222584, RPI.4610, squalamine lactate, SU5416, thalidomide, combretastatin, tamoxifen, COL-3, neovastat, BMS-275291, SU6668, anti-VEGF antibodies, Medi-522 (Vitaxin II)" CAI, Interleukin 12, IM862, amiloride, angiostatin, angiostatin KI-3, angiostatin KI-5, captopril, DL-alpha-difluoromethylornithine, DL-alpha-difluoromethylornithine HCl, endostatin, fumagillin, herbimycin A, 4-hydroxyphenylretinamide, juglone, laminin, laminin hexapeptide, laminin pentapeptide, lavendustin A, medroxyprogesterone, minocycline, placental ribonuclease inhibitor, suramin, thrombospondin, antibodies targeted against proangiogenic factors (for example, Avastin, Erbitux, Vectibix, Herceptin); low molecular weight tryrosine kinases inhibitors of proangiogenic growth factors (for example Tarceva, Nexavar, Sutent, Iressa); mTOR inhibitors (for example Torisel); alpha, beta and gamma interferon, IL-12, matrix metalloproteinase inhibitors (for example, COL3, Marimastat, Batimastat); ZD6474, SUI1248, vitaxin; PDGFR inhibitors (for example Gleevec); NM3 and 2- ME2; cilengitide, In a particular embodiment, the anti-angiogenic agent with which the compounds of the invention are conjugated is cilengitide.

The cytotoxic agents or anti-angiogenic agents conjugated with the compounds of the invention may be monoclonal antibodies.

The compounds of the invention may be used to carry photosensitizing compounds. These compounds have the advantage with respect to conventional cytotoxic compounds that they are inactive until they are locally activated in the target organ so that they reduce the secondary effects caused by the activity of these compounds in tissues other than the target tissue.

The terms "disease associated to an undesired cellular proliferation" and "disease associated to an undesired angiogenesis" have been described in detail in the context of therapeutic uses of the compounds of the invention and are applied in the same way to the conjugates in accordance with the present invention.

"Medicament" is understood to be a pharmaceutical composition comprising one of the compounds of the invention or a pharmaceutically acceptable salt thereof. In this specific case, the medicament must comprise at least another therapeutic agent useful in the treatment of the cancer.

In another aspect, the invention relates to a conjugate comprising a compound of the invention and a nanocarrier.

In the context of this invention, "nanocarrier" is understood to be any type of vehicle which transports, without limitation, genetic material (plasmid DNA, interference RNA, antisense oligonucleotides), ribozymes, antibodies, drugs or any other substance with pharmacological activity, and which is capable of releasing it in the suitable dose in the target cells. For this reason, said nanocarrier is conjugated with a compound according to the invention, which selectively targets the nanocarrier by active vectorization to the surface of the cells that express the αᵥβ₃ integrin, being useful for the treatment of diseases associated to an undesired cellular proliferation or to an undesired angiogenesis, such as, for example, cancer. The nanocarrier, in addition to being conjugated to the compound according to the invention, may also be conjugated with other molecules (antibodies, lectins, proteins, hormones, low molecular weight ligands) which facilitate the targeting of the drug.

The term "nanocarrier" in the present invention defines any carrier, irrespective of its size, for which reason said term includes both carriers whose size is of the order of µm and carriers whose size is of the order of nm.

The nanocarrier must have the suitable characteristics to protect the compound it transports. Thus, for example, a nanocarrier for nucleic acids has to provide the nucleic acid with an effective protection against degradation by nucleases, favouring their internalization to the interior of the cells and allow the release in functional form in the nucleus thereof. Examples of nanocarriers of the invention include, without limitation, synthetic release systems, generally cationic, such as liposomes, niosomes, dendrimers, lipoplexes, polyplexes, nanoparticles, nanoparticles of ceramic material, nanoparticles of calcium phosphate, magnetic nanoparticles, polymeric nanoparticles, solid lipid nanoparticles, nanocapsules, micelles, lipid drops in water and lipid emulsions, microcapsules, microspheres, etc.

Alternatively, the compounds of the invention may be used for the selective targeting of target tissues that express αᵥβ₃ integrins of nanoparticles that comprise therein cytotoxic agents or anti-angiogenic agents. Examples of this type of formulations are widely known for the person skilled in the art and include, without limitation, siRNA nanoparticles and pegylated polyethylenimine (PEI) wherein the RGD peptide ligand is bound to the distal end of the polyethylene glycol as described by Schiffelers RM et al., (Nucleic Acids Res., 2004, 32:e149) or the nanocarriers modified on their exterior with RGD ligands as described in US2010166808.

The conjugation of the compound of the invention with a cytotoxic agent, with an anti-angiogenic agent or with a nanocarrier is performed thanks to functional group R₁ of the thiazole or oxazole ring(s) of the compounds of the invention (Butler JD et al. 2010. Chem. Eur. J., in press DOI: 10.1002/chem.201001492). Any R₁ functional group suitable for this purpose may be used, but preferably said group is of amine, carboxy, alkynylalkyl or mercaptoalkyl type.

The nanocarriers useful in the invention also includes noble metal nanoparticles, and more specifically, gold nanoparticles, which show photothermal properties, which means that when activated in the presence of laser light (or microwave, radiofrequency or ultrasound) they give off heat, acting as real nano-heaters. This allows the selective laser photothermal treatment (hyperthermal) of the tumour cells, but also the release of active molecules on demand in specified parts of the body.

The gold nanoparticles may be produced with different sizes (1 nm - 150 nm) and forms (nanospheres, nanocylinders, nanocoated, nanocages, nanoparticles and SERS) and be easily functionalized with a wide range of ligands (antibodies, polymers, diagnostic probes, drugs, genetic material, ...), which makes it possible to prepare nanostructures useful for transport, vectorization and selective release of drugs and therapeutic macromolecules (peptides, proteins, etc.) and for gene therapy (vehiculization of plasmids, DNA, RNA, interference RNA, oligonucleotides, etc.) (Bhattacharya R and Mukherjee P. 2008. Advanced Drug Delivery Reviews, 60: 1289-1306; Chen PC et al. 2008. Nanotechnology, Science and Applications, I: 45-66; Jain PK et al. 2008. Accounts of Chemical Research, 41: 1578-1586). It also highlight the use of gold nanoparticles in creating «intelligent carrier systems» which make it possible to controls, in space and time, the release of the associated therapeutic compound, since said release is triggered by an internal biological stimulus (for example a variation in the glutathione concentration between the exterior and the interior of the cell) or by activation of an external stimulus (for example laser light).

The compounds of the invention conjugated with gold nanoparticles are useful in thermal phototherapy and selective vectorization of cancer, for the treatment of diseases associated to angiogenesis, as well as for use in drug release systems, therapeutic proteins and genetic material. Gold nanoparticles of 2 nm nucleus diameter are known which have been conjugated to approximately 7 molecules of a chemotherapeutic agent (Gibson JD et al. 2007. J. Am. Chem. Soc. 129: 11653-11661). Therefore, this invention also includes the conjugation of a metal nanoparticle with one or more molecules of the compounds of the invention. Said conjugated metal nanoparticle may be multifunctional and may also be conjugated with another /other chemotherapeutic or cytotoxic agent(s) different to the compounds of the invention, which may be a drug or an antibody; or with diagnostic agents that enable imaging diagnosis; or with polymers; or with optical probes. These compounds may be directly conjugated to the metal nanoparticles or by thiolated polyethylene glycol (PEG) bridges, since the functionalization of the gold nanoparticles is generally performed by thiol bonds, although they also show affinity for the amine, phosphite and bisulfite groups. Conjugation with metal nanoparticles increases the half-life time in the blood stream of the conjugated compound.

The conjugated metal nanoparticles have special application in phototherapy or hyperthermia treatment of cancer, where the gold nanoparticles functionalized with a compound of the invention that recognizes the αᵥβ₃ integrin specifically overexpressed on the surface of the tumour cells, specifically bind to said cells. Subsequently, the external irradiation of the tumour cells selectively marked with gold nanoparticles with a frequency laser adapted to the absorption maximum of said nanoparticles (10-50 nm) leads to a selective heating (40-80°C) which causes irreversible cell damage and the disorganization of the tissue components, and all of this at laser powers much lower than those required to destroy healthy cells. The nanoparticles may be designed to cross the cell membrane, become internalized in the cell and release the compound or molecule they contain and even be localized in the nucleus.

Dual gold nanoparticles may also be designed which integrate in the same diagnosis and therapy system (Loo C et al. 2005. Nanoletters, 4:709-711). Said nanoparticles allow the cancer to be diagnosed by molecular optical imaging (by dispersing the light in the near infrared) and they cause the selective destruction of sectorized carcinoma cells that express the αᵥβ₃ integrin by photothermal therapy. The gold nanoparticles may be detected in biological samples and tissues using a large variety of methods: electronic, dark field and laser scanning microscopy, optical tomography, Raman spectroscopy, X-ray image, etc.

Another type of nanocarriers are molecular cages such as those described in international patent applications WO/2003/096990 and WO/2008/048288.

In a preferred embodiment the nanocarrier of the invention contains a cytotoxic agent, an anti-angiogenic agent or a mixture of both.

In another aspect, the invention relates to the use of a conjugate comprising a compound of the invention and a nanocarrier containing a cytotoxic agent, an anti-angiogenic agent or a mixture of both for the preparation of a medicament for the treatment of a disease associated to an undesired cellular proliferation or to an undesired angiogenesis.

Alternatively, the invention relates to a conjugate comprising a compound of the invention and nanocarrier containing a cytotoxic agent, an anti-angiogenic agent or a mixture of both for use in the treatment of a disease associated to an undesired cellular proliferation or to an undesired angiogenesis.

Alternatively, the invention relates to a method for the treatment of a disease associated to an undesired cellular proliferation or to an undesired angiogenesis in a subject comprising the administration to said subject of a conjugate comprising a compound of the invention and a nanocarrier containing a cytotoxic agent, an anti-angiogenic agent or a mixture of both.

In the present document, the expression "carrier" relates to the compound according to the invention being directly or indirectly linked or associated to another therapeutic agent and is capable of targeting it towards the tumour target cells that express the αᵥβ₃ integrin.

In the context of the present invention, "another therapeutic agent" is understood to be a substance with therapeutic activity on the tumours other than the compounds of the present invention.

### OTHER ASPECTS OF THE INVENTION

Given that the compounds of the invention have high affinity for type αᵥβ₃ integrins, they may be used to purify integrins from a sample or for the detection of integrins in a biological sample.

In one aspect, the invention relates to a compound according to the invention for the detection of integrins in a sample.

In another aspect, the invention relates to a conjugate comprising a compound according to the invention and a solid support.

In another aspect, the invention relates to the use of a compound according to the invention or of a conjugate comprising a compound according to the invention and a solid support for the purification or detection of integrins in a sample.

Preferably, for their use in the purification of integrins, the compounds of the invention are used associated to a support or substrate. In principle, any type of support may be used in the methods of the invention, although it is preferable to use polymeric type supports such as Sephadex, dextran, water soluble polyamino acids, polyethylene glycol (PEG), polyglutamic acid (PGA), polylactic acid (PLA), polylactic-co-glycolic acid (PLGA), poly(D,L-lactide-coglycolide) (PLA/PLGA), poly(hydroxyalkylmethacrylamide), a polyglycerol, a polyamidoamine (PAMAM) and a polyethylenimine (PEI).

Typically, the purification of integrins using the compounds of the invention is carried out by a process comprising the steps of
(i) contacting the sample of the integrin to be purified with a compound of the invention immobilized on a support in suitable conditions for the bond between the compound and the integrin to take place;
(ii) washing the complexes formed in stage (i) to eliminate all those compounds of the sample which have been bound to the compoundsupport conjugate in non-specific form and
(iii) eluting the integrins that have been bound to the compound.

The integrin purification method of the invention may be carried out using any known protein purification method by affinity, including, for example affinity chromatography columns whose stationary phase is formed by the conjugates of the compounds according to the invention to a solid support.

Functional groups may be used that are introduced in the substituents of the thiazole and/or oxazole rings to immobilize the cyclic peptides of the invention in different materials such as polymers or stationary chromatographic phases. The most suitable functional groups for the immobilization of the cyclic peptides in said materials are amine, carboxy, alkynylalkyl or mercaptoalkyl. This allows the use of these compounds for analysis or diagnosis due to their capacity to bind to integrins.

Additionally, the detection of integrins in a biological sample may be carried out using known methods for the detection of compounds by the use of affinity reagents marked with a detectable compound.

Thus, in one embodiment, the invention contemplates an integrin detection method where the sample to be analysed is placed in contact with a support whereon a specific antibody for the integrin that one wants to detect has been immobilized in suitable conditions for the bond to occur between the integrin and the compound. Later, the complexes formed between the immobilized antibody of the invention and the integrin may be detected using the compounds marked with a detectable compound.

In another embodiment, the detection of integrins in a biological sample may be carried out using methods known for the detection of compounds by the use of affinity reagents marked with a detectable compound. Thus, the invention contemplates a method where the sample to be analysed is placed in contact with a support whereon the compound of the invention has been immobilized in suitable conditions for the bond to occur between the integrin and the compound. Later, the complexes formed between the immobilized compound of the invention and the integrin may be detected using an anti-integrin antibody. Said antibody may be marked with a detectable compound or be detected using methods known for a person skilled in the art such as:
- detection of the antibody using an anti-antibody antibody marked with a detectable compound,
- detection of the antibody if this is biotinylated using avidin conjugated to a detectable compound,
- detection of the antibody if this is biotinylated using an anti-biotin antibody conjugated to a detectable compound

"Detectable compound" is understood to be a compound whose nature allows its detection by enzymatic, radioactive or fluorescence methods. The detectable compound may be an enzyme, a radioactively labelled compound or a fluorochrome. The detectable compound may also be a biosensor. The detectable compounds are useful in the diagnosis and/or monitoring of diseases or states where there is an overexpression of the αᵥβ₃ integrin.

In another embodiment, the detection of integrins in a biological sample may be carried out using biosensors. "Biosensor" is understood to be a biochemicalelectronic device which makes it possible to identify, transform and quantify a biological event, and which is composed of the combination of a bioreceptor (biological component) and a transducer (detection method). In the context of this invention, the bioreceptor or recognition element of the αᵥβ₃ integrin, i.e. the compound of the invention, must be immobilized on a membrane or matrix, which is also fixed to the surface of the transducer. Among the techniques most used to immobilize the bioreceptor we have:
- physical adsorption: the recognition element binds to the matrix by ionic interactions, hydrogen bridges or Van der Waals forces. Therefore, the most suitable matrices are cellulose, silica gel, collagen, hydroxyapatite or acetate.
- trapping: the recognition element is retained physically in the interior cavities of the matrix. The most suitable matrices are gels (agar, nylon, starch, polyacrylamide) or rigid composite electrode matrices (graphiteteflon) or graphite-epoxy resin.
- Crossover: irreversible bonds occur between the recognition elements themselves and with the matrix by functional reagents. The reagents used may be hexamethylene-diisocyanate glutaraldehyde or 2,4-dinitrobenzene.
- Covalent bonds: covalent bonds occur of the recognition element with activated chemical groups of the matrix or directly of the transducer.

The transducer is the element that converts the variations of the physical or chemical properties produced by the interaction between the recognition element and the analyte in a signal that may be amplified, stored and recorded. The signal generated by the transducer in some case may not be directly interpreted and it is necessary to use software to process it. Transducers may be electrochemical, optical, piezoelectric (mass, gravimetric, acoustic), thermometric and nanomechanical. One type of transducer or another may be used depending on the nature of the interaction between the recognition element and the species of interest.

In the context of the present invention, "conjugate" is understood to be formed by a compound according to the invention linked, bound or associated to at least one second component. This second component may be a toxin, a detectable compound or a polymer.

Furthermore, the cyclic RGD peptides may also be useful in the coating of biomaterials to obtain bioactive implants. The coating of said materials with specific RGD cyclopeptides of the αᵥβ₃ integrin makes it possible to guarantee the bond between the implant and the tissue that expresses said integrin. They are of use, for example, in the coating of implants for osteoblasts that express the αᵥβ₃ integrin (Auernheimer J, Zukowski D, Dahmen C, Kantlehner M, Enderle A, Goodman SL, Kessler H (2005) ChemBioChem 6:2034; Elmengaard B, Bechtold JE, Søballe K (2005) Biomaterials 26:3521; Schliephake H, Scharnweber D, Dard M, Rossler S, Sewing A, Meyer J, Hoogestraat D (2002) Clin Oral Implants Res 13:312; Hersel U, Dahmen C, Kessler H (2003) Biomaterials 24:4385). Implants that may be coated with the cyclic peptides of the invention are bone implants, for example, dental implants. The material of said implants may be of metal, metal alloys, ceramic material and combinations thereof, preferably titanium and its metal alloys such as Ti-6Al-4V or Ni-Ti (with prior treatment of preferential oxidation), alloys based on Co-Cr or stainless steels, etc. For the coating of biomaterials with the cyclic peptides of the invention, the R₁ group of the thiazole or oxazole ring is used, preferably an amine, carboxy, alkynylalkyl or mercaptoalkyl group.

The invention is described below by the following examples, which must be considered as merely illustrative and in no case limiting of the scope of the present invention.

### EXAMPLES

The compounds obtained in the examples described below are identified using proton nuclear magnetic resonance data (¹H-NMR).

The operating frequency and the solvent used to record the spectrum are indicated in the spectrums. The positions of the signals are indicated in δ (ppm) and the signal of the protons of the solvent is taken as reference. The coupling constants (J) are expressed in Hertz. The number of protons is indicated between brackets of each signal by electronic integration and the type of signal using the following abbreviations: s (singlet), d (doublet), t (triplet), q (quartet), dd (doublet of doublets), ddd (doublet of doublet of doublets), td (triplet of doublets), m (multiplet).

The following abbreviations are used in the experimental part:
TEA: triethanolamine
DIPEA or DIEA: N,N-diisopropylethylamine
NMM: N-methylmorpholine
TFAA: trifluoroacetic anhydride
TFA: trifluoracetic acid
Py: pyridine
TIS or TIPS: triisopropylsilane
HPLC: high performance liquid chromatography
HPLC-MS: high performance liquid chromatography-mass spectrometry
DCM: dichloromethane
FCS: fetal calf serum
ELISA: enzyme-linked immunoabsorbent assay
BSA: bovine serum albumin
PBS: phosphate buffered saline
OD: optical density
DMF: N,N-dimethylformamide
HCTU: (2-(6-chloro-1H-benzotriazole-1-yl)-1,1,3,3-tetramethylhaminium hexafluorophosphate
HATU: O-(7-azabenzotriazole-1-yl)-N,N,N1,N'-tetramethyluronium hexafluorophosphate
THF: tetrahydrofuran
ACN: acetonitrile
MALDI-TOF-MS: Matrix assisted laser desorption ionisation time-of-flight mass spectrometry
PyAOP: (7-Azabenzotriazole-1-yloxy) tripyrrolidinophosphonium hexafluorophosphate
DIPCDI: Diisopropylcarbodiimide
RP-HPLC: reverse phase HPLC
AcOEt: ethyl acetate
AcOH: acetic acid
Thz: thiazole
EC₅₀: effective concentration 50
**Amino acids**
Gly (G): glycine
Arg (R): arginine
Asp (D): aspartic acid
Phe (F): phenylalanine
Val (V): valine
Ala (A): alanine
MeVal: N-methylated valine

A series of thiazole amino acids have been synthesized which have later been used to form a cyclic peptide structure mimicking the integrin antagonist structure of Cilengitide®. The bonding affinity of the compounds synthesized by the integrin receptors have been evaluated showing that said compounds have biological activity as antagonists of the αᵥβ₃ integrin.

### Material and methods

### Synthesis of cyclic peptides

**Cyclo[-Arg-Gly-Asp- 6 or 7 -Phe-Val-Ala-] (1 and 2).** *Resin loading. 2-*chlorotrityl chloride-resin (150 mg, 1.5mmol/g) was placed in a 20 ml polypropylene syringe fitted with a polyethylene filter disk. The resin was then washed with CH₂Cl₂ (5 × 0.5 min), and a solution of Fmoc-L-Gly-OH (334 mg, 1.125 mmol, 5 equiv) and DIEA (239 µL, 6.25 equiv) in CH₂Cl₂ (2.5 mL) was added. The mixture was then stirred for 15 min. Extra DIEA (239 µL, total 12.5 mmol) was added, and the mixture was stirred for an additional 45 min. The reaction was stopped by adding 3 × DCM/ MeOH/ DIEA (85:10:5) and stirring for 10 min. The Fmoc-L-Gly-O-resin product was subjected to the following washings/treatments with CH₂Cl₂ (3 × 0.5 min), DMF (3 x 0.5 min), piperidine and DMF (5 × 0.5 min). The loading was 0.50 mmol/g, as calculated by Fmoc determination.

*Peptide coupling.* Fmoc-L-Arg(Pbf)-OH (243 mg, 0.375 mmol, 5 equiv), Fmoc-L-Ala-OH (117 mg, 0.375 mmol, 5 equiv), Fmoc-L-Val-OH ( 127 mg, 0.375 mmol, 5 equiv) and Fmoc- L-Phe-OH (145 mg, 0.375 mmol, 5 equiv) were added sequentially to the above obtained *H*-L-Gly-O-resin using HCTU (155 mg, 0.375 mmol, 5 equiv), HOBt (50 mg, 0.375 mmol, 5 equiv) and DIEA (127 µL, 0.75 mmol, 10 equiv) in DMF (2.5 mL). In all cases, after 90 min of coupling, the ninhydrin test was negative. Removal of Fmoc group and washings were performed as described in general procedures. *N*-Alloc-thiazole **6** or **7** (92 mg, 0.375 mmol, 5 equiv) was coupled with HATU (143 mg, 0.375 mmol, 5 equiv), HOAt (51 mg, 0.375 mmol, 5 equiv) and DIEA (127 µL, 0.75 mmol, 10 equiv) for 90 min. This coupling was repeated twice in the same conditions. The Alloc group of the peptide resin was removed with Pd (PPh₃)₄ (9 mg, 0.0075 mmol, 0.1 equiv) in the presence of PhSiH₃ (92.5 µL, 0.75 mmol, 10 equiv) in DCM for 20 min. This deprotection was repeated three times in the same conditions. After washing, the resin was treated with dry THF (2mL) for 15 min. Meanwhile, Fmoc-L-Asp(tBu)-OH (154 mg, 0.375 mmol, 5 equiv) was added to a 68 mM solution of triphosgene in dry THF (1.15 equiv). *Sym*-collidine (99.5 µL, 0.75 mmol, 10 equiv) was added to the clear solution, upon which a precipitate of collidinium chloride was formed. DIEA (102 µL, 0.6 mmol, 8 equiv) was added to the resin, immediately followed by addition of the suspension. This coupling was repeated four times in the same conditions. The reaction mixture was stirred at 50°C during 48 h.

*Peptide cleavage.* Following Fmoc deprotection, the peptidyl-resin was treated with TFA-CH₂Cl₂ (1:99) (5 × 30 s). The filtrate was collected on H₂O (4 mL) and the H₂O was partially removed under reduced pressure. MeCN was then added to dissolve solid that formed during the removal of H₂O, and the solution was lyophilized to give 12 mg and 10 mg of the linear compounds **28** and **29** respectively with a purity of > 91 % as checked by HPLC (Column A, *R*ₜ 7.43 min and *R*ₜ 7.38 min respectively, linear gradient 35%-40% ACN in 15 min.)], which was used without further purification. MALDI-TOF-MS calculated for C₅₀H₇₁NnO₁₃S₂ 1098.29; found *m*/*z* 1099.29 [M + H]⁺, 1121.28 [M + Na]⁺, 1137.39 [M+K]⁺.

*Synthesis in solution. Cyclization.* The protected linear peptides **28** and **29** were dissolved in DMF (1 L, 10⁻⁴ M), and HOAt (9.6 mg, 0.07 mmol, 5 equiv), DIPEA (24 µL, 0.14 mmol, 10 equiv), and PyAOP (36.6 mg, 0.07 mmol, 5 equiv) were added. The mixture was stirred for 24 h at room temperature, and the course of the cyclization step was then checked by HPLC (Column A, *R*ₜ 11.67 min and *R*ₜ 10.70 min respectively, linear gradient 45%-55% ACN in 15 min.). The solvent was removed by evaporation under reduced pressure and the protected cycle **30** and **31** were used in the next step without further purification. MALDI-TOF-MS calculated for C₅₀H₆₉N₁₁O₁₂S₂ 1080.28; found *m*/*z* 1081.28 [M + H]⁺, 1103.27 [M + Na]⁺, 1119.38 [M + K]⁺.

*Side chain deprotection.* The protected cyclopeptides **30** and **31** (14.7 mg, 19.04 µmol) were treated with TFA-H₂0 (95: 5) during 1 h. The solvent was removed by evaporation under reduced pressure.

*Peptide purification.* The crude product was purified by HPLC (Symmetry C₈ 5 µm, 30 mm × 100 mm), gradient of MeCN (30% to 75% in 15 min) MeCN (+0.05% TFA) in water (+0.05% TFA), 20 mL/min, detection at 220 nm, to give the cyclopeptides **1** and **2** (4.5 mg, 5.8 µmol and 6.5 mg, 8.37 µmol, 7.7% and 12% yield respectively). The products were characterized by HPLC (Rₜ 8.99 min, and *R*ₜ 8.02 min Column A, respectively, linear gradient 0%-100% ACN in 15 min.) and by MALDI-TOF-MS: calculated for C₃₃H₄₅N₁₁O₉S 771.84; found *m*/*z* 772.84 [M + H]⁺, 794.83 [M + Na]⁺, 810.94 [M + K]⁺.

***Cyclo*-[Arg-Gly-Asp-Thz1X-] (3). General procedure for cyclopeptide synthesis.** *Solid phase synthesis:* The synthesis of the linear peptide HAsp(tBu)-XX-Arg(Pbf)-Gly-OH was performed using Fmoc-based solid phase peptide synthesis with 2-chlorotrityl chloride resin (2.0 g, 3.2 mmol).

*Resin loading:* Fmoc-Gly-OH (594 mg, 2.0 mmol) was attached to the resin with DIPEA in DCM at room temperature for 1.5 h. The remaining trityl groups were capped adding 0.5 mL of MeOH for 30 min. After that, the resin was filtered and washed with DCM (2x), DMF (2x). The loading of the resin was determined by titration of the Fmoc group (Chan WC and White PD. Fmoc Solid Phase Peptide Synthesis. Oxford University Press: New York, 2000). The final loading was 2.0 mmol/g. The Fmoc group was eliminated by treatment with 20% piperidine in DMF (2X10 min). The resin was washed with DMF (3x), DCM (3x).

*Peptide coupling:* Fmoc-Arg(Pbf)-OH (5.19 g, 8.0 mmol), DIPCDI (1.23 mL, 8.0 mmol) and HOBt (1.08 g, 8.0 mmol) were dissolved in DMF and added to the resin for 1.5 h. The end of the coupling was monitored by ninhydrin test (free amine group) (Kaiser E et al. Anal Biochem 1970, 34:595-598). The resin was filtered and washed with DMF (3X) and DCM (3X). The Fmoc group was eliminated with 20 % piperidine in DMF (2X10 min).

The coupling of the thiazole module was carried out with **8** (1.14 g, 3.0 mmol), PyAOP (1.56 g, 3.0 mmol) and DIPEA (1.02 mL, 6.0 mmol) in DMF for 1.5 h. The completion of the reaction was checked with the ninhydrin test. Finally the deprotection of the amine and coupling of the Fmoc-Asp(^{t}Bu)-OH were carried out under the same conditions of the second amino acid.

*Peptide cleavage:* The resin bound peptide was treated with 2% TFA in DCM (6 x 30 sec.) The resin was washed with DCM and the combined solution was evaporated under vacuum with Et₂O several times, furnishing the linear peptide **32** as a white solid. The peptide was used for the next step without purification.

HPLC (gradient 20 to 80% of CH₃CN in 15 min): t_{R}= 8.33 min. HPLC-MS (ES(+)): m/z 795.3.

*Synthesis in solution. Cyclization:* The product **32** (200 mg, 0.251 mmol) was dissolved in anhydrous DMF (50 mL, 5 mM), PyAOP (262 mg, 0.503 mmol) and DIPEA (213 µL, 1.255 mmol) were added. The reaction was monitored by HPLC. Once the reaction was finished, the DMF was evaporated under vacuum. The crude was dissolved in AcOEt and the solution was washed with NH₄Clₛₐₜ and Na₂CO₃ salt. The organic layer was collected, dried over Na₂SO₄, filtered and concentrated under vacuum. The peptide was purified by flash chromatography (CHCl₃/MeOH 8:2) furnishing the protected cyclic peptide **33** as a white solid (156 mg, XX%). HPLC (gradient 40 to 90% of CH₃CN in 15 min): t_{R}= 8.86 min. HPLC-MS (ES(+)): m/z 778.2

*Side chain deprotection*: The protected peptide **33** (125 mg, XX mmol), was treated with 25 mL of a solution of TFA/H₂O (95:5). After 3 h, the solvent was evaporated under vacuum and the residue was precipitated with Et₂O (4X). The Et₂O solution was discarded and the white solid was lyophilized to afford **3** 55 mg (XX%).

*Peptide purification.* The end product **3** was dissolved in 5 ml MilliQ water and it was filtered through a 0.2 µm filter. The cyclic peptide was purified by semipreparative RP-HPLC using acetronitrile (0.05% TFA)/water (0.1% TFA). The HPLC sample was vacuum concentred and transformed into the hydrochloride salt lyophilized with water with 0.05% HCI.¹H-NMR (500 MHz, H₂O:D₂O-d² 9:1, 278 K): δ = 9.29 (t, N*H* Gly), 9.20 (d, J = 7.24 Hz, N*H* Asp), 8.90 (t, J = 5.89/5.89 Hz, N*H* Thz), 8.46 (d, J = 8.93 Hz, N*H* Arg), 7.79 (s, C*H* Thz), 7.22 (t, J = 5.39/5.39 Hz, N*H*_{ε} Arg), 4.75 (m, C*H*_{α} Arg), 4.63 (m, C*H*_{α} Asp), 4.04 (dd, J = 3.35/14.90 Hz, C*H*_{α} Gly), 3.82 (dd, J = 6.69/14.96 Hz, C*H*_{α} Gly), 3.17 (m, C*H*_{2δ} Arg), 2.89 (m, C*H*_{2β} Asp), 1.92 (m, C*H*_{β} Arg), 1.82 (m, C*H*_{β} Arg), 1.63 (m, C*H*₂ γ Arg). HPLC (gradient 0 to 20% of CH₃CN in 15 min): t_{R}= 10.52 min. HRMS (EIS) m/z calculated 468.1540 (C₁₇H₂₄N₈O₆S) found 469.16099 (M+H)⁺.

***Cyclo*-[Arg-Gly-Asp-Thz2X-] (4)**. The cyclopeptide **4** was prepared according to the process followed for **3** and using bithiazole **9** (XX mg, YY mmol) instead of **8**. The linear peptide **34:** HPLC (gradient 0 to 100% CH₃CN in 15 min.): t_{R} = 10.34 min, HPLC-MS (ES(+)): m/z 877.81. The protected peptide **35:** HPLC (gradient 0 to 100% CH₃CN in 15 min.): t_{R} = 13.91 min, HPLC-MS (ES(+)): m/z 860.54. The final peptide **4:** ¹H-NMR (500 MHz, H₂O:D₂O-d² 9:1, 298 K): δ = 8.93 (S_{broad}, N*H* Gly), 8.82 (d, J = 7.62 Hz, N*H* Asp), 8.75 (t, J = 5.69/5.69 Hz, N*H* Thz), 8.51 (d, J = 7.62 Hz, N*H* Arg), 8.05 (s, C*H* Thz¹), 7.50 (s, C*H* Thz²), 7.19 (t, J = 5.38/5.38 Hz, N*H*_{ε} Arg), 4.13 (dd, J = 5.82/14.24 Hz, C*H* Gly), 3.87 (dd, J = 5.96/15.69 Hz, C*H* Gly), 3.21 (m, C*H*_{2δ} Arg), 2.94 (m, C*H*_{2β} Asp), 1.95 (m, C*H*_{β} Arg), 1.87 (m, C*H*_{β} Arg), 1.68 (m, C*H*_{2γ} Arg). HPLC (gradient 10 to 25% of CH₃CN in 15 min): t_{R} = 8.73 min. HRMS (EIS) m/z calculated 551.1369 (C₂₀H₂₅N₉O₆S₂) found 552.14392 (2M+2H)⁺.

***Cyclo*-[Arg-Gly-Asp-Thz3X-] (5).** The cyclopeptide **5** was prepared according to the process for **3** and using trithiazole **10** (XX mg, YY mmol) instead of **8.** The linear peptide **36:** HPLC (gradient 20 to 80% of CH₃CN in 15 min.): t_{R} = 7.60 min, HPLC-MS (ES(+)): m/z 961.23. The protected peptide **37:** HPLC (gradient 20 to 80% of CH₃CN in 15 min.): t_{R} = 13.13 min, HPLC-MS (ES(+)): m/z 944.3. The final peptide **5:** HPLC (gradient 10 to 30% CH₃CN in 15 min): t_{R} = 8.26 min. HRMS (EIS) m/z calculated 634.1199 (C₂₃H₂₆N₁₀O₆S₃) found 635.12683 (2M+2H)⁺. ¹H-NMR (500 MHz, DMSO-d⁶, 298 K): δ = 9.21 (t, J = 5.4, N*H* Gly), 8.72 (m, N*H* Asp + N*H* Thz), 8.37 (s, C*H* Thz¹), 7.96 (d, J = 9.2, N*H*_{α} Arg), 7.77 (s, C*H* Thz²), 7.68 (t, J = 6.0, N*H*_{ε} Arg), 7.23 (s, C*H* ThZ³) 4.83 (dd, J = 14.3, 8.5, C*H*_{α} Arg), 4.72 (dd, J = 16.3, 6.6, C*H* Thz), 4.59 (m, C*H* Thz + C*H*_{α} Asp), 3.89 (d, J = 11.5, C*H* Gly), 3.59 (d, J = 9.7, C*H* Gly), 3.13 (dd, J = 12.6, 6.3, C*H*_{2δ} Arg), 2.81 (dd, J = 16.3, 4.3, C*H*_{β} Asp), 2.58 (dd, J = 16.5, 8.7, C*H*_{β} Asp), 1.82 (m, C*H*_{β} Arg), 1.71 (m, C*H*_{β} Arg), 1.49 (m, C*H*_{2γ} Arg).

**Cilengitide.** The cilengitide was prepared according to the method described in Dechantsreiter MA et al. (J Med Chem 1999, 42:3033-3040). ¹H-NMR (500 MHz, H₂O:D₂O-d² 9:1, 298 K): δ = 8.55 (d, J = 8.06 Hz, N*H* Asp), 8.37 (d, J = 7.28 Hz, N*H* Arg), 8.13 ( d, J = 9.19 Hz, N*H* Phe), 7.97 (m, N*H* Gly), 7.34 (m, 2H, C₆H₅ Phe), 7.26 (m, 3H, C₆H₅ Phe), 7.22 (t, J = 5.53/5.53 Hz, N*H*_{ε} Arg), 5.19 (dd, J = 8.58/16.02 Hz, C*H*_{α} Phe), 4.56 (dd, J = 7.45/- Hz, C*H*_{α} Asp), 4.34 (d, J = 10.89 Hz, C*H*_{α} MeVal), 4.12 (dd, J = 7.80/14.63 Hz, C*H* Gly), 3.95 (dd, J = 6.84/15.33 Hz, C*H*_{α} Arg), 3.54 (dd, J = 3.37/14.60 Hz, C*H* Gly), 3.20 (m, C*H*_{2δ} Arg), 3.02 (m, C*H*_{2β} Phe), 2.88 (s, C*H*₃ MeVal), 2.84 (dd, J = 7.26/16.68 Hz, C*H*_{β} Asp), 2.63 (dd, J = 7.60/16.54 Hz, C*H*_{β} Asp), 2.06 (m, C*H*_{β} Val), 1.91 (m, C*H*_{2β} Arg), 1.57 (m, C*H*_{2γ} Asp), 0.88 (d, J = 6.55 Hz, C*H*₃ Val¹), 0.56 (d, J = 6.49 Hz, CH₃ Val²).

**Biological experiments.** Cells were grown in DMEM High Glucose (GIBCO, Ref: 31966), supplemented with 10% FCS (GIBCO, 10106-169) at 37°C and 5% CO2. Cells were expanded when they reached 90% confluence. Non-tissue culture treated ELISA plates were coated at 4°C with the appropriate concentration of the ligand (depending on the cell line). The coating solution was discarded and the wells were blocked with blocking solution (1.5% BSA; 60 minutes at 37°C). The blocking solution was discarded by flicking and serial dilutions of the compounds were plated in duplicates. Immediately, harvested cells were plated at a given concentration (20000-25000 /well for HUVEC and DAOY and 50000 for HT-29) to the same plate. Plates were incubated for 60-90 min at 37°C to allow cell adhesion and spreading on the ligands. Plates were washed three times with PBS and 100 µl/well of Hexosaminidase Substrate solution was added. Plates were incubated at 37°C during 3 hours and then stop solution was added. Adsorption was read at OD 405nm.

Each plate contains positive and negative controls and peptides were tested as duplicates. Each assay has been repeated at least twice and the adhesion inhibition EC₅₀ was calculated, when possible, using the Prism-4 software based on the sigmoidal dose-response (variable slope) equation.

### Results and discussion

### Chemistry

In an initial phase, preliminary studies were carried out to investigate the introduction of 2-amino-4-thiazole carboxylic acid and 2-amino-5-thiazole carboxylic acid in a linear peptide chain and after promoting the corresponding cyclization to obtain cyclic RGD peptides (compounds 1 and 2) that incorporate the heterocyclic amino acids in the Cilengitide® structure.

The synthesis of the first series of thiazole amino acids was performed by cyclocondensation protocols, based on Hantzsch reaction (interaction of thiourea with ethyl bromopyruvate and 2-chloro-3-oxopropanoate). In this way, both ethyl 2-amino-4-thiazolcarboxylate (Ciufolini MA and Shen YC. J Org Chem 1997, 62:3804-3805; Kumar et al. Heteroc Chem 2002, 8:521-530) and ethyl 2-amino-5-thiazolecarboxylate (Kennedy AR et al. Acta Cryst Sec E 2001, 57:o832-o833; Zhao R et al. Tetrahedron Lett 2001, 42:2101-2102) were obtained in good yields. After protection of the amine group and hydrolysis of the ester, building blocks **6** and **7** are ready to use in solid phase peptide synthesis (see Diagram 1).

**Diagram 1.** Synthesis of the N-protected thiazole amino acids **6** and **7.**

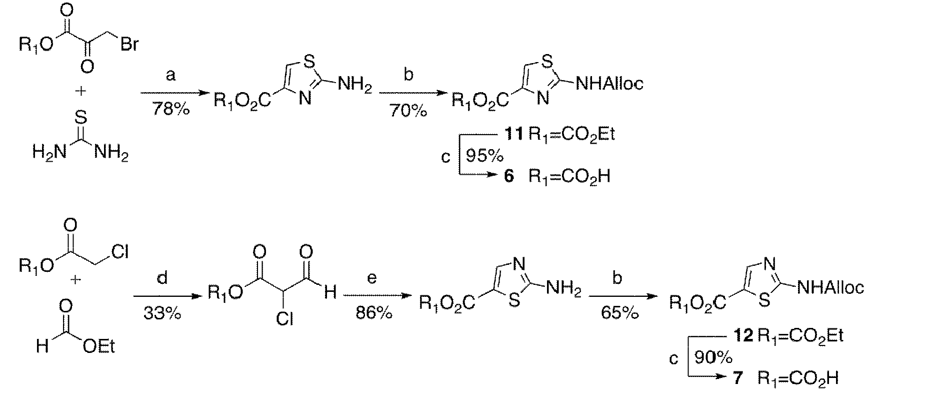

Reagents and conditions: (a) EtOH, Δ, (b) allyl chloroformate, TEA, (c) NaOH in EtOH, (d) NaOEt, (e) thiourea in EtOH, Δ.

New derivatives were designed where a methylene bridge was introduced between the amine and the thiazole ring in position 2. The resulting amino acids substitute the sequence *D*-Phe-*N*Me-Val of the cilengitide structure by an amino acid based on 1, 2 or 3 thiazole rings to obtain compounds **3, 4** and **5.**

The synthesis of the second series of thiazole amino acids was devised through a stepwise route adapting the methods described in the literature (Koerber-Pie K and Massiot G. J Het Chem 1995, 32:1309-1315). The iterative protocol allowed a convenient multigram synthesis of the Fmoc-protected amino acids **8, 9** and 10 which were obtained in good yields (see Diagram 2).

**Diagram 2.** Synthesis of the N-protected thiazole amino acids **8, 9** and **10.**

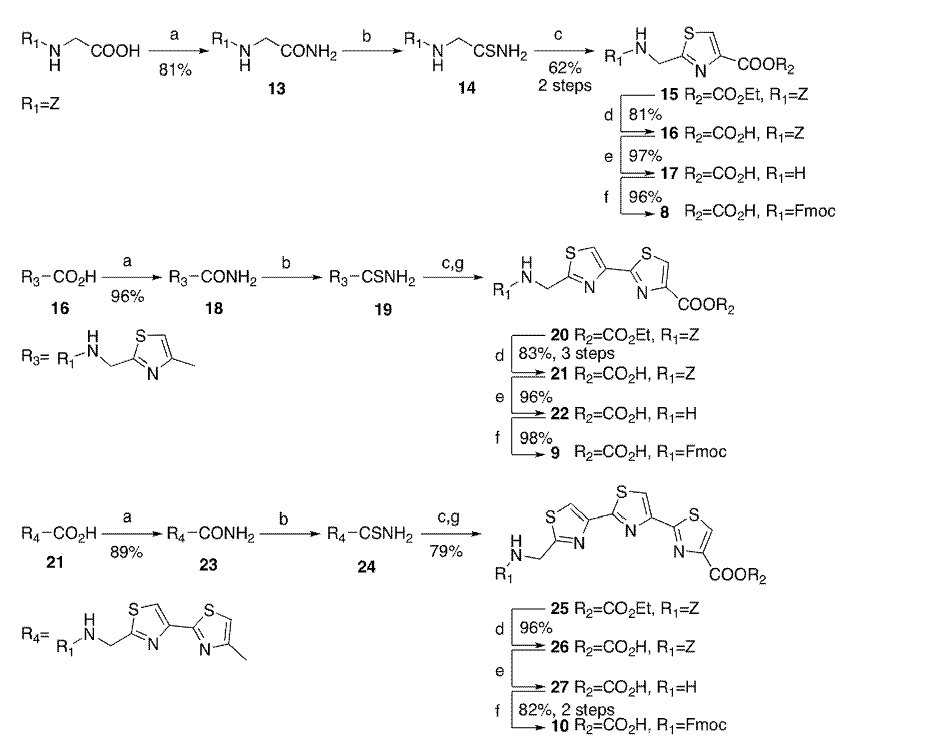

Reagents and conditions: (a) isobutyl chloroformate, NMM, NH_{3aq}, (b) Lawesson reagent, (c) ethyl bromopyruvate, NaHCO₃, (d) LiOH, (e) HBr in AcOH, (f) Fmoc-Cl, Na₂CO₃, (g)TFAA, Py.

The linear peptides were synthesized by solid-phase method using Fmoc/tBu chemistry, and the cyclization and the final deprotection were performed in solution. The protective groups of the side chains of Asp and Arg are orthogonal with the concentrations of TFA since the 2-chlorotrityl chloride resin allows the release of the protected peptides. The final deprotection was performed without TIS as scavenger, because when we used it, we detected the corresponding cyclopeptides with thiazoline residue (M+2 peaks were detected in the HPLC-MS analysis). To avoid this, the final deprotection was carried out with TFA/H₂O (95:5).

Finally, compounds 1, 2, 3, 4, 5 and cilengitide as comparative compound were obtained.

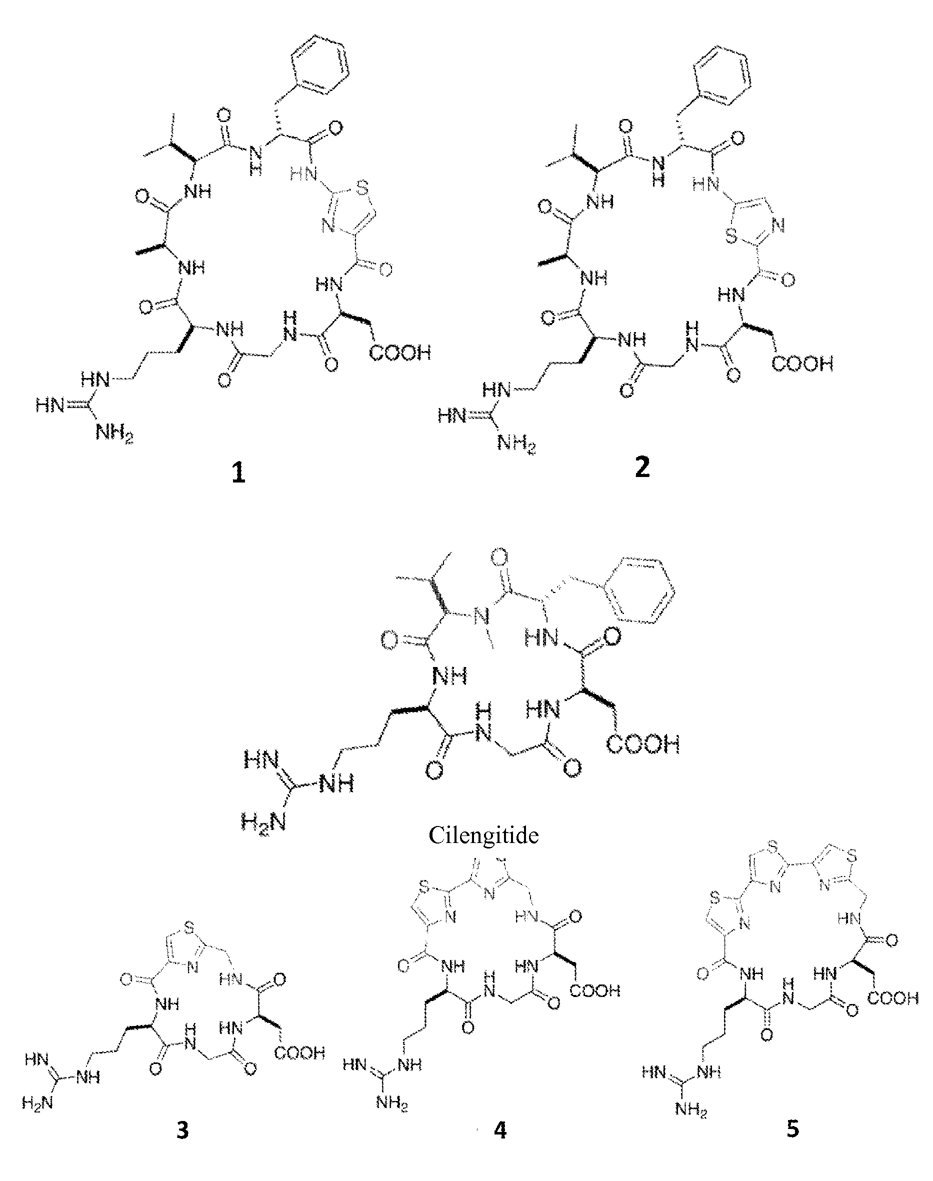

### Biology

The different RDG derivates based on thiazole-amino acid structures were tested for their biological activity such as inhibition of cell adhesion using three different cell systems and three integrin ligands for each cell line (vitronectin (VN), fibrinogen (FB) and fibronectin (FN) and collagen (COL)). The cell lines used for the study were: DAOY human glioblastoma cells and HUVEC human umbilical vein endothelial cells as primary cell culture, which overexpresses in both members, αᵥβ₃ and αᵥβ₅, of the αᵥ-integrin family, and HT29 human colon adenocarcinoma, which overexpresses in both members, αᵥβ₅ and αᵥβ₆, of the αᵥ-integrin family.

In the first series of experiments compounds **1-5** were tested using HUVEC cell line showing that only compounds **4** and **5** were active. The next experiments using DAOY and HT-29 cell lines were carried out only with the most active compounds **4** and **5.**

Cilengitide has been the reference control in all the assays performed in this study. Its activity in the different cell systems correlates well with the existing data.

In the experiments using VN as integrin ligand, both HUVEC and DAOY cells use αᵥβ₃ and αᵥβ₅ to adhere to VN; while HT29 cells use only αᵥβ₅ to adhere to VN. In contrast, in the experiments using FB as integrin ligands, both HUVEC and DAOY cells use αᵥβ₃ to adhere to FB, and the experiments using FN and COL were to control that the compounds could no inhibit the independent cell adhesion.

In this scenario **4** and **5** show blocking activity in the HUVEC/DAOY to VN system but with marked less potency than Cilengitide. On the other hand, both **4** and **5** show the same activity as Cilengitide to block αᵥβ₃ mediated cell adhesion to FB, clearly suggests that **4** and **5** have both different activity and different affinity to the αᵥβ₅ integrin.

Finally, the data analysis of the HT29-VN cell system provides the definitive information to conclude that both **4** and **5** have no blocking activity against αᵥβ₅.

As expected, none of the compounds, including the reference one (Cilengitide) would inhibit cell adhesion to αᵥ-integrin independent cell adhesion (FN and COL coatings) (Table 1).

**Table 1: Activity for VN and FB**

| | **Adhesion inhibition - EC-50 (µM)** | | | | | |
|---|---|---|---|---|---|---|
| | Cell lines | VN Coating [µg/ml] | **3** | **4** | **5** | **Cilengitide** |
| **VN** | HUVEC (αᵥβ₃/αᵥβ₅) | 0.75 | 31.13 | 7.46 | 63.80 | 0.56 |
| | DAOY (αᵥβ₃ /αᵥβ₅) | 0.5 | ND | 5.71 | 6.80 | 0.068 |
| | HT-29 (αᵥβ₅) | 4.0 | ND | 53.16 | 59.05 | 2.24 |
| **FB** | HUVEC (αᵥβ₃) | 4.0 | 368.4 | 4.95 | 33.15 | 4.03 |
| | DAOY (αᵥβ₃) | 5.0 | ND | 0.082 | 1.86 | 0.059 |

### Conclusions

Compound **3** is clearly an inactive compound in all cell systems used in the study. Compounds **4** and **5** have similar activities in all cell systems, except in the case of cell adhesion to FB, which is only mediated by the αᵥβ₃ integrin. In this case, and analysing the adhesion of the HUVEC and DAOY cells to FB, compound **4** showed greater activity than **5** and both were selective antagonists of the αᵥβ₃ integrin Furthermore, compound **4** has a similar potency to cilengitide.

## Claims

1. A compound of general formula (I): 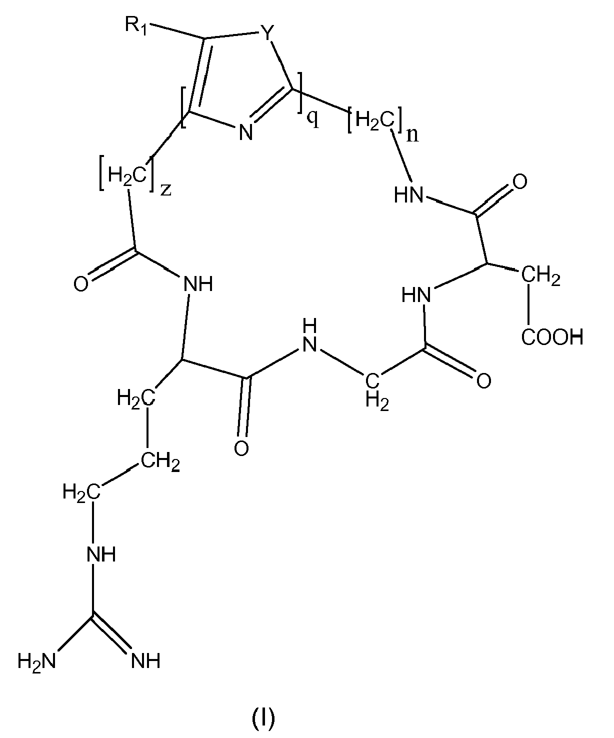 its stereoisomers, mixtures thereof and/or its pharmaceutically acceptable salts wherein:
Y is independently selected for each heterocycle of O and S;
R₁ is independently selected for each heterocycle of the group formed by H, OH, halogen, alkoxyl, aryloxyl, alkyl, aryl, acyl, amine, cycloalkyl, carboxy, alkynylalkyl and mercaptoalkyl;
n is a whole number selected from 0 and 2;
q is a whole number selected from 2 and 3 and
z is a whole number selected from 0 and 1.

2. A compound according to the preceding claim wherein the compound has formula (II) or formula (III):

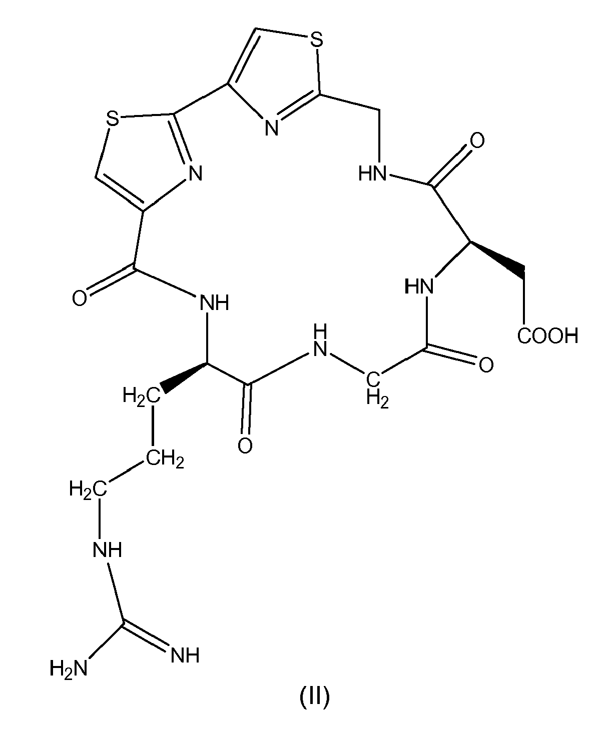

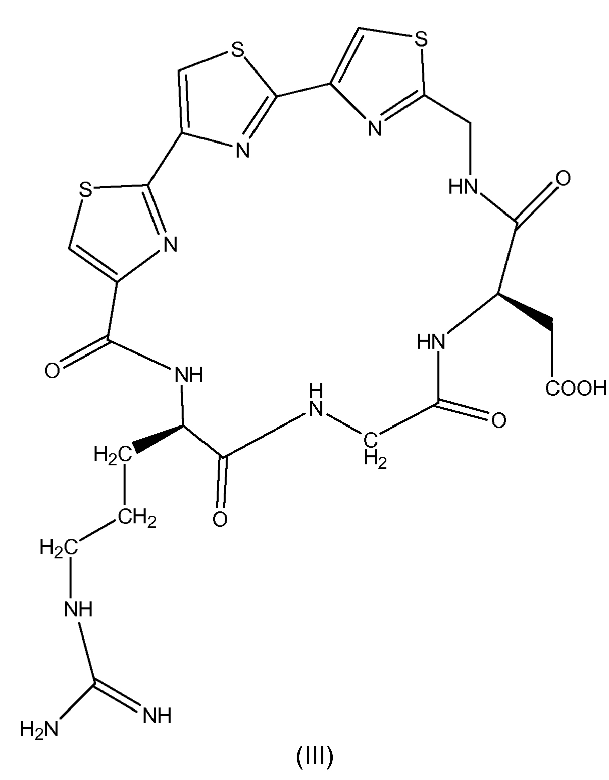

3. Process for obtaining a compound of general formula (I), its stereoisomers, mixtures thereof, or its pharmaceutically acceptable salts as defined in any of claims 1 or 2, comprising the cyclization between the amino-terminal end and the carboxyl-terminal end of a linear peptide selected from the group of
Arg-Gly-Asp-X
Gly-Asp-X-Arg
Asp-X-Arg-Gly and
X-Arg-Gly-Asp
wherein X is an amino acid based on thiazole and/or oxazole nuclei of formula (IV) 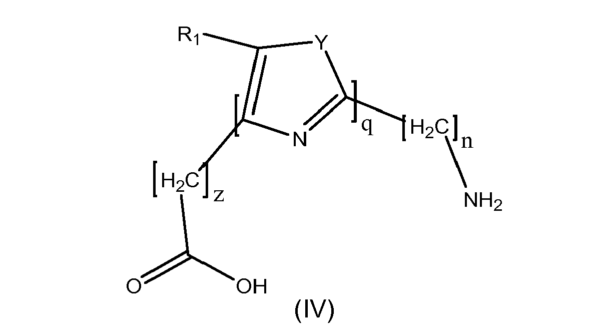 wherein:
Y is independently selected for each heterocycle of O and S;
R₁ is independently selected for each heterocycle of the group formed by H, OH, halogen, alkoxyl, aryloxyl, alkyl, aryl, acyl, amine, cycloalkyl, carboxy, alkynylalkyl and mercaptoalkyl,
n is a whole number selected from 0 and 2,
q is a whole number selected from 2 and 3 and
z is a whole number selected from 0 and 1.

4. Process according to claim 3, wherein the linear peptide cyclized is
Asp-X-Arg-Gly

5. Process according to any of claims 3 or 4, wherein X is an amino acid selected from the group of the amino acid of formula (V) and of the amino acid of formula (VI):

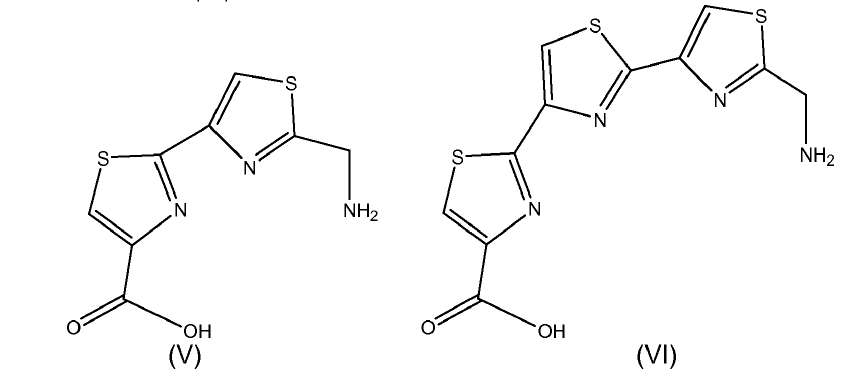

6. A linear peptide selected from one of the following formulas:
Arg-Gly-Asp-X
Gly-Asp-X-Arg
Asp-X-Arg-Gly and
X-Arg-Gly-Asp
wherein X is an amino acid based on thiazole and/or oxazole nuclei of formula (IV) 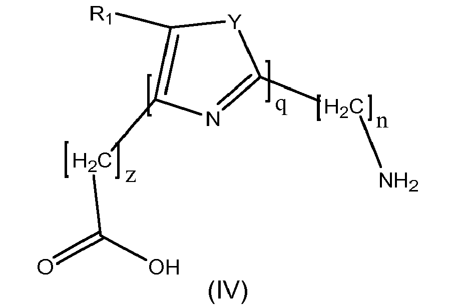 wherein:
Y is independently selected for each heterocycle of O and S;
R₁ is independently selected for each heterocycle of the group formed by H, OH, halogen, alkoxyl, aryloxyl, alkyl, aryl, acyl, amine, cycloalkyl, carboxy, alkynylalkyl and mercaptoalkyl;
n is a whole number selected from 0 and 2;
q is a whole number selected from 2 and 3;
and z is a whole number selected from 0 and 1 and
wherein the residues of Arg and Asp are optionally protected.

7. Pharmaceutical composition comprising a pharmaceutically effective quantity of at least one compound of general formula (I), its stereoisomers, mixtures thereof and/or its pharmaceutically acceptable salts, according to any of claims 1 or 2, and at least one pharmaceutically acceptable excipient.

8. Use of a compound according to any of claims 1 or 2 for the preparation of a medicament for the prevention and/or the treatment of a disease associated to an undesired cellular proliferation, a disease associated to an undesired angiogenesis, metastasis, restenosis, osteoporosis, an inflammatory disease, proliferative diabetic retinopathy and/or acute renal insufficiency.

9. A conjugate comprising a compound according to any of claims 1 or 2, and a second component selected from the group of
(i) a cytotoxic agent and
(ii) an anti-angiogenic agent

10. Use of a conjugate according to claim 9, for the preparation of a medicament for the treatment or prevention of a disease associated to an undesired cellular proliferation or to an undesired angiogenesis.

11. A conjugate comprising a compound according to any of claims 1 or 2, and a diagnostic imaging agent.

12. Use of a conjugate according to claim 11, for the preparation of a diagnostic agent.

13. A conjugate comprising a compound according to any of claims 1 or 2 and a nanocarrier.

14. A conjugate according to claim 13, wherein the nanocarrier contains a cytotoxic agent, an anti-angiogenic agent or a mixture of both.

15. Use of a conjugate according to claim 14, for the preparation of a medicament for the treatment of a disease associated to an undesired cellular proliferation or to an undesired angiogenesis.
